# EUROPÄISCHE PATENTSCHRIFT

(11) **EP 4 239 336 B1**
(45) Veröffentlichungstag und Bekanntmachung des Hinweises auf die Patenterteilung: **10.01.2024**
(21) Anmeldenummer: 22160018.2
(22) Anmeldetag: 03.03.2022
(51) Int. Cl.: G01N 33/543

(54) **VERFAHREN ZUM HERSTELLEN EINER MIKROTITERPLATTE MIT FÄNGERMOLEKÜLBESCHICHTUNGEN UND MIKROTITERPLATTE**
METHOD OF MANUFACTURING A MICROTITRATION PLATE WITH CAPTURE MOLECULAR COATINGS AND MICROTITRATION PLATE
PROCÉDÉ DE FABRICATION D'UNE PLAQUE DE MICROTITRAGE DOTÉE DE REVÊTEMENTS À MOLÉCULES DE CAPTURE ET PLAQUE DE MICROTITRAGE

(43) Veröffentlichungstag der Anmeldung: 06.09.2023
(73) Patentinhaber: Euroimmun Medizinische Labordiagnostika AG, 23560 Lübeck (DE)
(72) Erfinder: DIBBERN, Jannike, 23560 Lübeck (DE); HAEFKE, Mathias, 23560 Lübeck (DE); GOERL, Monique, 23560 Lübeck (DE); SCHRÖDER, Dieter, 23560 Lübeck (DE); MEYER, Wolfgang, 23560 Lübeck (DE); SCHEPER, Thomas, 23560 Lübeck (DE); OTT, Anthonina, 23560 Lübeck (DE); BRECH, Arne, 23560 Lübeck (DE); FEIRER, Christian, 23560 Lübeck (DE); RATEIKE, Martin, 23560 Lübeck (DE)

(56) Entgegenhaltungen:
- DE-A1-102013 008 468
- US-A1- 2015 011 437
- US-B2- 9 366 668
- SUJATHA RAMACHANDRAN ET AL: "A Rapid, Multiplexed, High-Throughput Flow-Through Membrane Immunoassay: A Convenient Alternative to ELISA", DIAGNOSTICS, Bd. 3, Nr. 2, 2. April 2013 (2013-04-02), Seiten 244-260, XP055549823, DOI: 10.3390/diagnostics3020244
- June Mai ET AL: , 1. Juli 2015 (2015-07-01), XP055743991, Gefunden im Internet: URL:https://cdn.shopify.com/s/files/1/0718 /6639/files/NatureApplicationNote_MemAssay .pdf?4 [gefunden am 2020-10-26]
- A. ALRASHOUDI ABDULELAH ET AL: "Fabrication of a Lateral Flow Assay for Rapid In-Field Detection of COVID-19 Antibodies Using Additive Manufacturing Printing Technolog", INTERNATIONAL JOURNAL OF BIOPRINTING, Bd. 7, Nr. 4, 23. August 2021 (2021-08-23) , Seite 399, XP055932370, ISSN: 2424-7723, DOI: 10.18063/ijb.v7i4.399 Gefunden im Internet: URL:https://ijb.whioce.com/index.php/int-j -bioprinting/article/viewFile/399/265>
- ANFOSSI LAURA ET AL: "Multiplex Lateral Flow Immunoassay: An Overview of Strategies towards High-throughput Point-of-Need Testing", BIOSENSORS, Bd. 9, Nr. 1, 26. Dezember 2018 (2018-12-26), Seiten 1-14, XP055855313, CH ISSN: 2079-6374, DOI: 10.3390/bios9010002
- "Bio-Dot SF Microfiltration Apparatus - Instruction Manual - Catalog Number 170-6542, 170-6543", 2 April 2013 (2013-04-02), Biorad pages 1-24, * the whole document *

## Beschreibung

Die Erfindung betrifft ein Verfahren zum Herstellen einer Mikrotiterplatte mit Fängermolekülbeschichtungen. Die Erfindung betrifft ferner eine Mikrotiterplatte mit Fängermolekülbeschichtungen in Form von Blotlinien-Segmenten in wenigstens einem Well. Die Erfindung betrifft ferner ein Verfahren, in welchem eine erfindungsgemäße Mikrotiterplatte für eine Inkubation verwendet wird. Die Erfindung betrifft ferner ein digitales Bildverarbeitungsverfahren.

Fängermoleküle im Sinne dieser Anmeldung sind Antigenmoleküle oder Antikörpermoleküle. Somit können im Sinne dieser Anmeldung Fängermoleküle auch als Antigene oder Antikörper bezeichnet werden. Im Sinne dieser Anmeldung umfasst der Begriff Antigene auch sogenannte Allergene.

Auf dem Gebiet der medizinischen Labordiagnostik sind unterschiedliche Testsysteme bekannt, mit denen das Vorhandensein spezieller Antikörper in Patientenproben überprüft wird. Da Antikörper im Körper eines Patienten nach einer viralen oder bakteriellen Infektionskrankheit gebildet werden, können mit derartigen Tests akute oder aber bereits von dem Patienten durchgemachte Krankheiten diagnostiziert werden. Ebenso können bei Auswahl entsprechend geeigneter Antigene Autoimmunerkrankungen oder Allergien diagnostiziert werden.

Im Bereich der In-vitro-Diagnostik werden zur Diagnoseerstellung vielfach Immunblotstreifen, insbesondere Line-Blot-Streifen, eingesetzt. Die jeweils benötigten Immunblotstreifen werden üblicherweise in hierfür vorgesehene Inkubationsrinnen eingelegt, in denen sie mit den erforderlichen Reagenzien sowie der Patientenprobe inkubiert werden. Nach einer korrekten Testdurchführung erscheinen bei positiv getesteten Patientenproben auf den Immunblotstreifen spezielle Verfärbungen, die sogenannten Banden, wodurch insbesondere die Bindung von Antikörpern an die auf den Immunblotstreifen vorgesehenen Antigene nachweisbar ist.

Die Auswertung der inkubierten Immunblotstreifen kann entweder durch eine Sichtprüfung erfolgen oder aber die Immunblotstreifen werden mit Hilfe automatisierter oder zumindest teilautomatisierter Systeme ausgewertet. In diesem Zusammenhang ist das System EU-ROBlotOne von der Firma EUROIMMUN Medizinische Labordiagnostika AG bekannt, das Aufnahmen von inkubierten Immunblotstreifen macht, während sich die Streifen noch in den Inkubationsrinnen einer Inkubationswanne befinden. Die entsprechenden Aufnahmen werden ferner gegebenenfalls digitalisiert, an eine Datenauswerteeinheit übertragen und dort mit Hilfe einer speziellen Laborsoftware ausgewertet. Bei der Auswertung der Immunblotstreifen wird zur Vorbereitung der Erstellung eines Diagnosevorschlags zunächst das Auftreten der Banden sowie die Verfärbung der einzelnen Banden im Vergleich zum Hintergrund ermittelt. Bei stark positiven Proben kommt es regelmäßig zu einer starken Verfärbung auf den Immunblotstreifen, während lediglich schwach positive Proben oftmals nur schwache und somit ggf. schwer auszuwertende Signale liefern.

Beim Immunblot-Prinzip werden Antigene auf Membranen als Festphase eingesetzt, um spezifische Antikörper mithilfe einer enzymatischen Farbreaktion in Patientenproben nachzuweisen. Die Durchführung erfolgt manuell, teil- oder vollautomatisiert. Enthält eine Probe spezifische Antikörper, so binden diese an die membrangebundenen Antigene. In einem weiteren Schritt wird ein Konjugat hinzugegeben, welches vorzugsweise ein alkalische-Phosphatase(AP)-markierter Antikörper ist, welcher wiederum an die spezifischen Antikörper bindet. Das Konjugat katalysiert mit einem anschließend hinzuzugegebenen Substrat eine Farbreaktion. Das Substrat ist vorzugsweise Nitroblautetrazoliumchlorid/5-Brom-4-chlor-3-indolylphosphat (NBT/BCIP). Sind spezifische Antikörper in der Patientenprobe vorhanden, so erscheint eine Verfärbung bzw. eine dunkle Linie an der jeweiligen Antigenposition als sogenannte Bande. Die Intensität der entstehenden Färbung ist dabei proportional zur Antikörperkonzentration in der Probe. Solche Färbungen können dann vorzugsweise mittels digitaler Bildverarbeitung erfasst und ggf. ausgewertet werden.

Lineblotstreifen mit mehreren, unterschiedlichen Antigenen sind sogenannte Multiparameter-Linienblots und ermöglichen die Erstellung umfangreicher kombinierter Antikörperprofile auf einem Teststreifen. Als antigenhaltige Festphase werden Membranstreifen mit mehreren aufgereinigten, biochemisch charakterisierten Antigenen oder Antigenextrakten verwendet, die als dünne parallele Linien bzw. Blotlinien auf dem Lineblotstreifen bzw. dem Membranstreifen aufgetragen sind.

Ferner ist es für diagnostische Fragestellungen eine mögliche Aufgabe, ein Vorhandensein von Antigenen in Patientenproben festzustellen. Hierzu können also als Fängermolekülbeschichtungen Antikörperbeschichtungen auf einem Line-Blot Streifen vorgesehen sein, um eine Bindung von Antigenen an Antikörper festzustellen.

Ferner können als eine sogenannte Serum-Kontrolle und/oder Konjugatkontrolle als Fängermolekülbeschichtungen in Form von Antikörperbeschichtungen vorgesehen sein.

Für eine diagnostische Fragestellung kann es daher vorteilhaft sein, mehrere solche Lineblotstreifen mit unterschiedlichen Fängermoleküllinien unterschiedlicher Fängermolekültypen bereitzustellen, vorzugsweise auch zu prozessieren bzw. zu inkubieren und besonders bevorzugt auch auszuwerten. Im Sinne dieser Anmeldung kann eine Fängermoleküllinie auch als eine Blotlinie bezeichnet werden, welche insbesondere Antigene oder Antikörper aufweist.

Aus S. Ramachandran 2013, "A Rapid, Multiplexed, High-Throughput Flow-Through Membrane Immunoassay: A Convenient Alternative to ELISA" ist ferner bekannt ein vakuumbetriebenes Durchflussmembran-Immunoassy im 96-Well Format zur Multiplexanwendung mit vier verschiedenen Bindungspartner pro Well.

Aus dem Dokument EP2427267 B1 ist es ferner bekannt, eine Mikrotiterplatte aus Glas mit Wells bereitzustellen, wobei der Wellboden eine Beschichtung mit darin befindlichen Löchern aufweist, in welche direkt Antigenspots eingebracht werden. Derartige Mikrotiterplatten weisen dann mehrere Vertiefungen bzw. Wells auf, in welchen jeweils mehrere Antigenspots vorhanden sind. Es kann dann im Zuge einer Inkubation mit einer Patientenprobe, einem Konjugat und einem Substrat eine Bindung von Antikörpern an die auf dem Wellboden befindlichen Antigenspots nachgewiesen werden.

Aufgabe der vorliegenden Erfindung ist es, eine Mikrotiterplatte mit Fängermolekülbeschichtungen bereitzustellen, welche besonders einfach bzw. praktikabel in ihrer Herstellung ist.

Ein weiterer Vorteil kann darin liegen, eine Mikrotiterplatte bereitzustellen, deren Fängermolekülbeschichtungen mittels eines digitalen Bildverarbeitungsverfahrens effizient und zuverlässig optisch ausgewertet werden kann. Die erfindungsgemäße Aufgabe wird gelöst durch ein erfindungsgemäßes Verfahren zum Herstellen einer Mikrotiterplatte mit Fängermolekülbeschichtungen, eine erfindungsgemäße Mikrotiterplatte sowie ein erfindungsgemäßes Verfahren, in welchem eine erfindungsgemäße Mikrotiterplatte verwendet wird, als auch einem erfindungsgemäßen digitalen Bildverarbeitungsverfahren.

Aus dem Stand der Technik ist es also bekannt, eine Mikrotiterplatte in der Weise mit Antigen zu beschichten, dass mittels einer Dispensierkapillare bzw. einer Dispensiernadel Antigenpunkte bzw. Antigenspots direkt auf einen beschichteten Wellboden eines Wells aufgebracht werden.

Hierfür ist es notwendig, für eine räumliche Verteilung mehrerer Antigenpunkte, welche für eine Multiparameteranalyse jeweilige, individuelle Antigentypen aufweisen sollen, während des sogenannten Spotting-Vorgangs die Dispensierkapillare in einer XY-Ebene präzise oberhalb des Wellbodens zu bewegen und zu positionieren.

Soll später im Zuge einer automatisierten Bildverarbeitung bzw. Bildanalyse in sicherer Weise festgestellt werden, für welche jeweiligen Antigenspots mit jeweiligen, individuellen Antigentypen sich jeweilige Bindungen von Antikörpern in Patientenproben ergeben haben, so muss also eine hohe Anforderung bezüglich der Positioniergenauigkeit der Dispensierkapillare in XY-Richtung insbesondere während des Bewegens innerhalb des Wells gestellt werden. Eine derartige Herstellung einer Mikrotiterplatte mit Antigenbeschichtungen als eine Ausführungsform von Fängermolekülbeschichtungen auf einem Wellboden kann daher produktionstechnisch aufwendig und auch fehleranfällig sein.

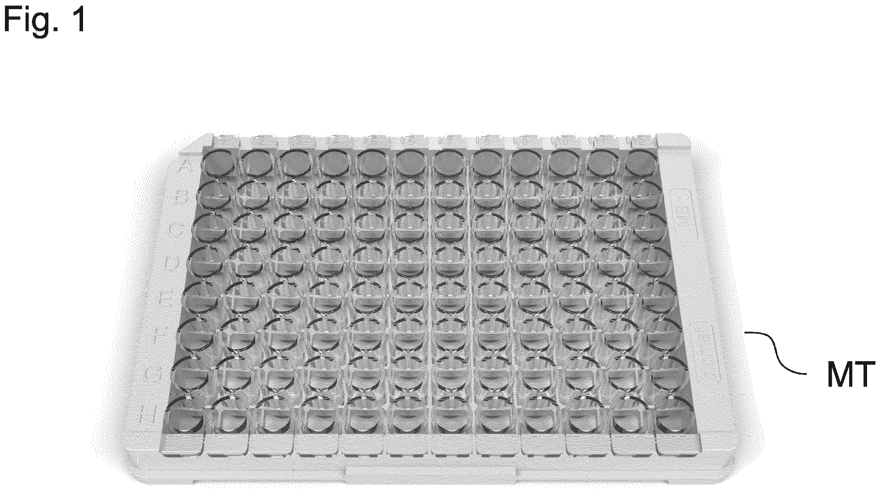

Eine beispielhafte Mikrotiterplatte MT ist in der Figur 1 gezeigt.

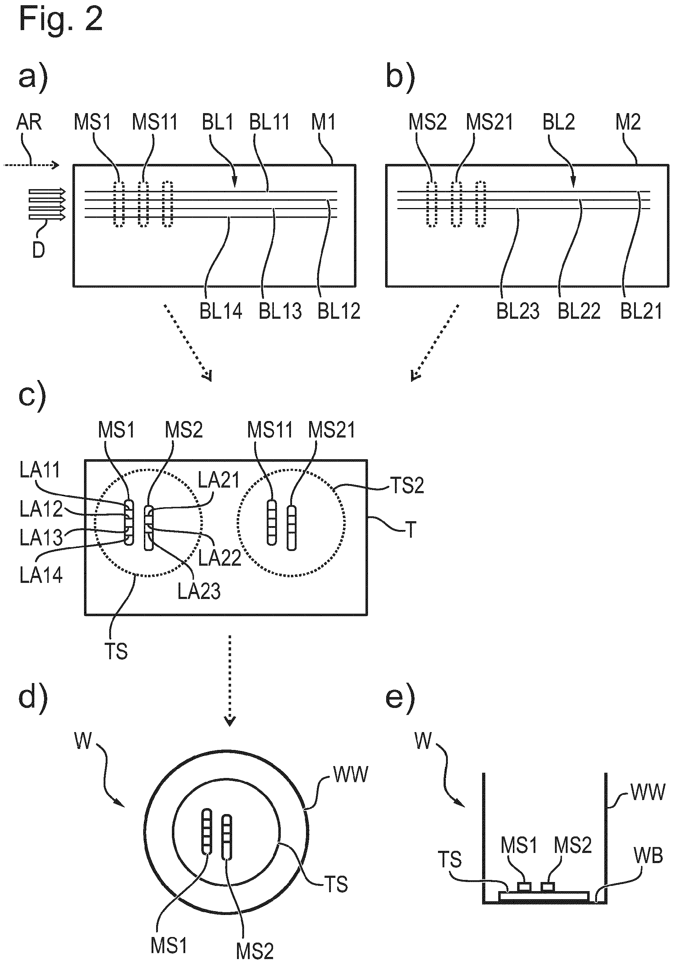

Die Figuren 2A bis 2E illustrieren das erfindungsgemäße Verfahren zum Herstellen einer Mikrotiterplatte mit Fängermolekülbeschichtungen. Das Verfahren umfasst verschiedene Schritte.

Wie in Figur 2A dargestellt, wird eine erste Trägermembran M1 bereitgestellt. Wie in der Figur 2B dargestellt, wird ferner eine zweite Trägermembran M2 bereitgestellt.

Gemäß der Figur 2A erfolgt ein Beschichten der ersten Trägermembran M2 mit jeweiligen ersten Blotlinien BL11, ... , BL14, welche auch als eine erste Menge von Blotlinien BL1 bezeichnet werden kann. Die jeweiligen ersten Blotlinien BL11, ..., BL14 weisen jeweils einen jeweiligen Fängermolekültypen auf. Diese jeweiligen Fängermolekültypen können unterschiedlich sein.

Das Beschichten der Trägermembran M1 mit den Blotlinien BL1 erfolgt mittels Bewegen mehrerer Dispensierkapillare bzw. Dispensiernadeln D entlang einer Auftragsrichtung bzw. Beschichtungsrichtung AR. Hierfür werden die Dispensiernadeln D in der Auftragsrichtung AR über die Membran M1 geführt. Die Bewegung ist insbesondere eine Linearbewegung.

In analoger Weise erfolgt gemäß der Figur 2B ein Beschichten der zweiten Trägermembran M2 mit jeweiligen zweiten Blotlinien BL21, ..., BL23, welche auch als Menge von Blotlinien BL2 bezeichnet werden kann.

Es erfolgt ferner ein Heraustrennen eines ersten Membransegmentes MS1, welches in der Figur 2A mittels einer gestrichelten Linie indiziert ist, aus der ersten Trägermembran M1. Das Membransegment MS1 weist dann jeweilige Linienabschnitte LA11, ..., LA14 der jeweiligen ersten Blotlinien BL11, .., BL14 auf, wie in der Figur 2C dargestellt.

Im Sinne dieser Anmeldung kann ein Linienabschnitt auch als ein Liniensegment bzw. ein Blotliniensegment bezeichnet werden.

Ferner erfolgt ein Heraustrennen eines zweiten Membransegmentes MS2 aus der zweiten Trägermembran M2. Wie aus der Figur 2C ersichtlich, weist das zweite Membransegment MS2 jeweilige Linienabschnitte LA21, ..., LA23 der jeweiligen zweiten Blotlinien BL21, ..., BL23 auf.

Es können vorzugsweise weitere Membransegmente MS11 bzw. MS21 aus den Membranen M1 bzw. M2 herausgetrennt werden.

Wie die Figur 2C illustriert, erfolgt ferner ein Positionieren und Fixieren des ersten Membransegmentes MS1 auf einer Trägerschicht T sowie ein Positionieren und Fixieren des zweiten Membransegmentes MS2 auf der Trägerschicht T. Vorzugsweise erfolgt ferner ein Positionieren und Fixieren des Membransegmentes MS11 auf der Trägerschicht T sowie ein Positionieren und Fixieren des Membransegmentes MS21 auf der Trägerschicht T.

Vorzugsweise erfolgt ferner ein Positionieren und Fixieren des Membransegmentes MS11 auf der Trägerschicht T sowie ein Positionieren und Fixieren des Membransegmentes MS21 auf der Trägerschicht T in auf der Trägerschicht insbesondere auf einem weiteren Bereich TS2, welcher bevorzugt ein weiteres Trägerschichtsegment bildet.

Die gestrichelte Kreislinie TS in der Figur 2C indiziert ein Trägerschichtsegment, welches aus der Trägerschicht herausgetrennt wird und welches das erste und das zweite Membransegment MS1, MS2 aufweist.

Wie die Figur 2D illustriert, erfolgt ferner ein Positionieren und Fixieren des Trägerschichtsegmentes TS in einem Well W einer Mikrotiterplatte. Figur 2D zeigt die Well-Wand WW sowie das Trägerschichtsegment TS aus einer Aufsicht von oben, so dass von der Öffnung des Wells W her die Trägerschicht TS sichtbar ist.

Die Figur 2E illustriert in der seitlichen Schnittansicht des Wells W die Lage des Trägerschichtsegmentes TS mit den darauf befindlichen Membransegmenten MS1, MS2 oberhalb des Wellbodens WB und zwischen den Well-Wänden WW.

Das Positionieren und Fixieren des Trägerschichtsegmentes TS in dem Well W erfolgt insbesondere in der Weise, dass die Membransegmente MS1, MS2 von der Öffnung OF des Wells her sichtbar sind.

Das Fixieren der Membransegmente bzw. das Fixieren des Trägerschichtsegmentes kann vorzugsweise als ein mechanisches Fixieren bezeichnet werden. Die Membransegmente können vorzugsweise auch als Trägermembransegmente bezeichnet werden.

Vorzugsweise erfolgt nach dem Beschichten der ersten Trägermembran M1 mit den ersten Blotlinien BL1 ein Blockieren der ersten Blotlinien BL1 auf der Trägermembran M1 mit einem ersten Blocking-Buffer. Vorzugsweise erfolgt ferner nach dem Beschichten der zweiten Trägermembran M2 mit den zweiten Blotlinien BL2 ein Blockieren der zweiten Blotlinien BL2 auf der zweiten Trägermembran M2 mit einem zweiten Blocking-Buffer, welcher insbesondere ein anderer Blocking-Buffer als der erste Blocking-Buffer sein kann. Es sind also vorzugsweise die ersten Liniensegmente LA11, ..., LA13 mit dem ersten Blocking-Buffer blockiert und es sind die zweiten Liniensegmente mit dem zweiten Blocking-Buffer blockiert.

Zur Darlegung eines oder mehrerer Vorteile des erfindungsgemäßen Verfahrens erfolgen nun genauere Ausführungen.

Das erfindungsgemäße Verfahren birgt den Vorteil, dass eine Mehrzahl an Fängermolekülbeschichtungen oberhalb des Wellbodens des Wells einer Mikrotiterplatte eingebracht bzw. positioniert werden können, ohne dass eine Dispensierkapillare direkt innerhalb des engen Raumes eines Wells positioniert und bewegt werden muss.

Dadurch, dass das Trägerschichtsegment TS in einem einzigen Schritt in dem Well oberhalb des Wellbodens positioniert werden muss, gibt es nur einen einzigen Arbeitsschritt, bei welchem eine besonders genaue Positionierung eines Werkzeuges innerhalb des Innenraums der Vertiefung des Wells erreicht werden muss. Das Positionieren der Dispensierkapillaren kann außerhalb des Wells vorgenommen werden, so dass während dieses Arbeitsschrittes mehr Arbeitsraum zur Verfügung steht.

Insbesondere kann durch das erfindungsgemäße Verfahren eine Verwendung von Standardlinienbeschichtungsvorrichtungen, welche die Dispensierkapillare aufweisen, erfolgen, ohne spezielle Dispensiervorrichtungen zum Aufbringen von Fängermolekülbeschichtungen auf einem Wellboden bereitstellen zu müssen.

Es ergibt sich also eine räumliche Freiheit von Werkzeugen, welche beim Beschichten der Membranen, dem Heraustrennen der Membransegmente sowie dem Auftragen der Membransegmente auf die Trägerschicht als auch dem Heraustrennen der Trägerschichtsegmente aus der Trägerschicht verwendet werden, da alle entsprechenden Schritte außerhalb des Innenraums des Wells erfolgen können. Daher kann eine Zusammenstellung von mehreren Fängermolekülbeschichtungen in Form von mehreren Blotliniensegmenten verteilt auf mehrere Membransegmente modular erfolgen und dies insbesondere außerhalb des Innenbauraums des Wells.

Ferner können unterschiedliche Kombinationen von Fängermolekülbeschichtungen bzw. Blotliniensegmenten für verschiedene diagnostische Fragestellungen und eben für verschiedene Wells relativ einfach konfiguriert bzw. hergestellt werden. Es können beispielsweise durch Austauschen der zweiten Trägermembran, welche die zweiten Blotlinien aufweist, durch eine dritte Trägermembran mit darauf befindlichen dritten Blotlinien, welche andere Fängermolekültypen als die der zweiten Blotlinien aufweisen, andere Fängermolekültyp-Kombinationen auf dem Trägerschichtsegment erreicht werden können.

Ferner ist eine Chargenkontrolle der Fängermoleküle nach Aufbringen der Blotlinien auf eine Membran in einfacher Weise möglich, da beispielsweise aus der Membran M1 ein Testmembransegment MS11 herausgetrennt werden kann, welches in einer separaten Prozessierungsvertiefung bzw. Blotwanne prozessiert bzw. inkubiert werden kann, um die Qualität der Fängermoleküle der Blotlinien BL1 zu überprüfen. Es muss also für eine Chargenkontrolle keine Prozessierung bzw. Inkubation der Fängermolekülbeschichtungen direkt in einem Well erfolgen, bei welcher dann später eine gesamte Mikrotiterplatte entsorgt werden müsste. Stattdessen kann das Membransegment MS11 separat außerhalb der Mikrotiterplatte prozessiert und ausgewertet werden.

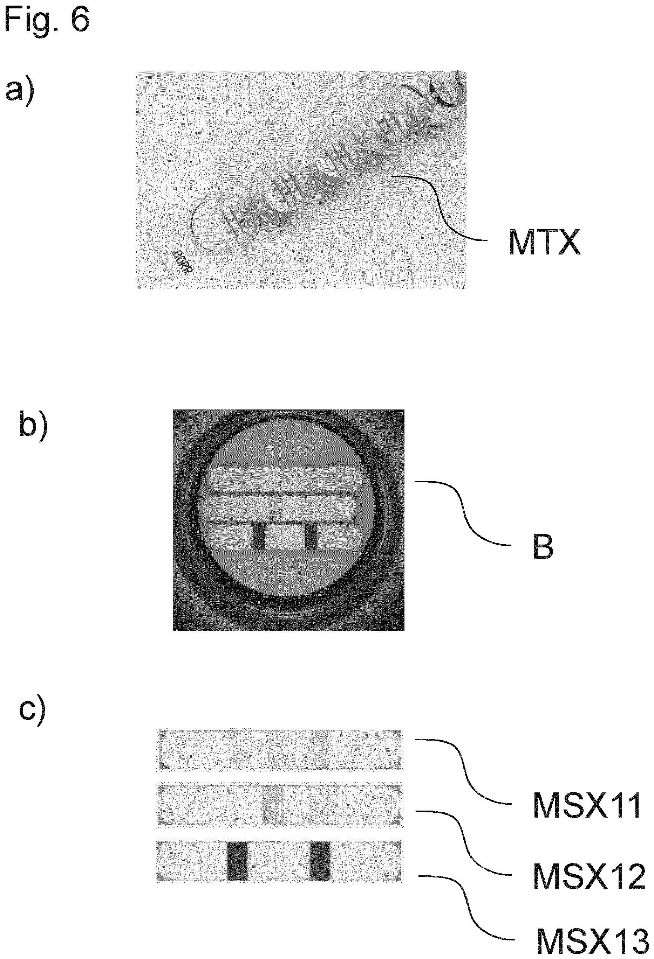

Im Sinne dieser Anmeldung ist eine Mikrotiterplatte eine Einheit, welche mehrere Wells bzw. Vertiefungen aufweist, welche mechanisch miteinander fest verbunden sind. Hierbei kann vorzugsweise die Anordnung der einzelnen Wells in einer Matrix-Anordnung gegeben sein, wie in der Mikrotiterplatte MT aus der Figur 1. Alternativ kann eine Anordnung mehrerer Wells entlang nur einer Ausbreitungsrichtung bzw. einer Linearanordnung gegeben sein, wie in der Figur 6A als eine Mikrotiterplatte MTX gezeigt.

In dem Fall, dass eine Beschichtung der Blotlinien BL1, BL2 auf den Trägermembranen M1, M2 erfolgt, kann ferner aufgrund des hier vorgeschlagenen besonders bevorzugten Verfahrens eine Blockierung der ersten Blotlinien BL1 auf der Membran M1 mit einem anderen Blockierungs-Buffer geschehen als für die zweiten Blotlinien BL2 auf der Membran M2. Hierdurch können also individuelle Blockierungsflüssigkeiten je nach Fängermolekültypen verwendet werden, welches eine höhere Stabilität der jeweiligen Blotlinien ermöglicht.

Für eine Auswertung eines Bildes eines Wells, wie in der Figur 2D gezeigt, durch ein digitales Bildbearbeitungsverfahren, ist eine präzise Positionierung von Fängermolekülbeschichtungen, beispielsweise in Form der Blotlinien-Abschnitte LA1, ..., LA14 sowie LA21, ..., LA23, notwendig. Eine solche Positionierung kann durch die Werkzeuge außerhalb des Wells sehr gut aufgrund des bereitstehenden Agitationsraums der Werkzeuge gewährleistet werden. Lediglich für den Arbeitsschritt des Einbringens des Trägerschichtsegmentes TS in den Innenraum des Wells W muss dann in einem engen Arbeitsbereich innerhalb des Wells W ein einzelnes Werkzeug präzise positioniert werden.

Vorteilhafte Ausführungsformen der Erfindung sind Gegenstand der abhängigen Ansprüche und werden in der folgenden Beschreibung unter teilweise Bezugnahme auf die Figuren näher erläutert.

Die ersten Blotlinien sind vorzugsweise parallel zueinander verlaufende Blotlinien. Die zweiten Blotlinien sind vorzugsweise parallel zueinander verlaufende Blotlinien.

Insbesondere erfolgt das Heraustrennen der Membransegmente mittels Herausschneiden, bevorzugt mittels Ausstanzen. Das Ausstanzen erfolgt besonders bevorzugt mittels eines Stanzmessers.

Insbesondere weisen die Membransegmente jeweils eine jeweilige längliche Ausdehnung, ferner eine jeweilige Längsachse entlang der länglichen Ausdehnung sowie ferner jeweils eine jeweilige Querachse senkrecht zur jeweiligen Längsachse auf.

Insbesondere weist das Trägerschichtsegment eine kreisförmige Grundfläche auf, wobei ferner das Positionieren der Membransegmente und das Heraustrennen des Trägerschichtsegmentes so erfolgt, dass die länglichen Membransegmente in der Weise auf dem Trägerschichtsegment positioniert sind, dass
- die Membransegmente mit ihrer jeweiligen Längsachse jeweils senkrecht zu einer Mittelachse der kreisförmigen Grundfläche des Trägerschichtsegmentes positioniert sind,
- ferner das erste Membransegment mit seiner Querachse zu der Mittelachse um eine erste Distanz versetzt positioniert ist
- und ferner das zweite Membransegment mit seiner Querachse zu der Mittelachse um eine zweite Distanz versetzt positioniert ist, welche sich von der ersten Distanz unterscheidet.

Die Mittelachse ist insbesondere eine gemeinsame Mittelachse.

Insbesondere erfolgt das Fixieren der Membransegmente auf der Trägerschicht mittels einer zwischen den Membransegmenten und der Trägerschicht befindlichen bzw. dort aufgetragenen Klebeschicht.

Insbesondere erfolgt das Fixieren des Trägerschichtsegmentes in dem Well auf dem Boden des Wells mittels einer zwischen dem Wellboden und dem Trägerschichtsegment befindlichen bzw. auf dem Trägerschichtsegment aufgebrachten Klebeschicht.

Insbesondere erfolgt das Fixieren des Trägerschichtsegmentes in dem Well mittels Einklemmen des Trägerschichtsegmentes innerhalb der inneren Seitenwand des Wells.

Vorgeschlagen wird ferner eine Mikrotiterplatte mit wenigstens einem Well mit Fängermolekülbeschichtungen, aufweisend ein Trägerschichtsegment, welches oberhalb eines Wellbodens in dem Well positioniert und fixiert ist, ferner auf dem Trägerschichtsegment zwei oder mehr positionierte und fixierte Trägermembransegmente welche jeweils mehrere Blotliniensegmente aufweisen, wobei die jeweiligen Blotliniensegmente jeweilige Fängermolekültypen aufweisen.

Insbesondere sind die Blotliniensegmente eines Trägermembransegmentes parallel zueinander verlaufende Blotliniensegmente.

Insbesondere umfasst die Mikrotiterplatte oberhalb des Trägerschichtsegmentes wenigstens eine Klebeschicht, auf welcher die Trägermembransegmente fixiert sind.

Insbesondere besteht die Klebeschicht aus mehreren Klebeschichtabschnitten, wobei ferner insbesondere die Klebeschichtabschnitte jeweils Abschnitte einer doppelseitigen Klebefolie sind.

Insbesondere ist das Trägerschichtsegment mittels einer weiteren Klebeschicht auf dem Wellboden des Wells fixiert, wobei vorzugsweise die weitere Klebeschicht eine doppelseitige Klebefolie ist.

Insbesondere weisen die Trägermembransegmente jeweils eine jeweilige längliche Ausdehnung, ferner jeweils eine jeweilige Längsachse entlang der länglichen Ausdehnung sowie ferner jeweils eine jeweilige Querachse senkrecht zur jeweiligen Längsachse auf, wobei ferner das Trägerschichtsegment eine kreisförmige Grundfläche aufweist und wobei ferner die länglichen Trägermembransegmente in der Weise auf dem Trägerschichtsegment positioniert sind, dass die Trägermembransegmente mit ihrer jeweiligen Längsachse jeweils senkrecht zu einer Mittelachse MA der kreisförmigen Grundfläche des Trägerschichtsegmentes positioniert sind, ferner das erste Trägermembransegment mit seiner Querachse zu der Mittelachse um eine erste Distanz versetzt positioniert ist und ferner das zweite Trägermembransegment mit seiner Querachse zu der Mittelachse um eine zweite Distanz versetzt positioniert ist, welche sich von der ersten Distanz unterscheidet.

Vorgeschlagen wird ferner ein Verfahren umfassend die Schritte: Bereitstellen einer erfindungsgemäßen Mikrotiterplatte aufweisend die Trägermembransegmente, Inkubieren der Trägermembransegmente mit einer Patientenprobe, einem Konjugat und einem Substrat. Das Verfahren umfasst vorzugsweise die weiteren Schritte: Erfassen eines Bildes, welches das Trägerschichtsegment und die Trägermembransegmente repräsentiert, Identifizieren des ersten Trägermembransegmentes und des zweiten Trägermembransegmentes in dem Bild, Bestimmen jeweiliger Bindungsmaße, welche eine jeweilige Bindung von Antikörpern oder Antigenen an jeweilige Blotliniensegmente indizieren, sowie Ausgeben der jeweiligen Bindungsmaße.

Vorgeschlagen wird ferner: Bereitstellen eines Datensatzes, welcher für jeweilige räumliche Lagen jeweiliger Querachsen jeweilige Membransegmenttypen indiziert, Identifizieren des ersten Trägermembransegmentes und des zweiten Trägermembransegmentes in dem Bild unter Verwendung des Datensatzes, Bereitstellen von Teilbildern, welche die jeweiligen Membransegmente repräsentieren.

Im Folgenden wird die Erfindung anhand spezieller Ausführungsform ohne Beschränkung des allgemeinen Erfindungsgedankens anhand der Figuren näher erläutert. Dabei zeigen:
- Figur 2A: eine erste Trägermembran mit ersten Blotlinien,
- Figur 2B: eine zweite Trägermembran mit zweiten Blotlinien,
- Figur 2C: eine Trägerschicht mit darauf positionierten und fixierten Membransegmenten,
- Figur 2D: die Trägerschicht, welche positioniert und fixiert in einem Well ist,
- Figur 2E: eine Seitenansicht des in dem Well positionierten und fixierten Trägerschichtsegmentes,
- Figur 3A-3C: Schritte einer ersten Ausführungsform des erfindungsgemäßen Verfahrens,
- Figuren 3D-E: bevorzugte Ausführungsformen von Wells einer Mikrotierplatte,
- Figur 4A-4C: Schritte einer zweiten Ausführungsform des erfindungsgemäßen Verfahrens,
- Figuren 4D-E: weitere bevorzugte Ausführungsformen von Wells einer Mikrotierplatte,
- Figur 5A: eine detaillierte Darstellung einer bevorzugten Ausführungsform eines Trägerschichtsegmentes mit Membransegmenten,
- Figur 5B: eine weitere bevorzugte Ausführungsform eines Trägerschichtsegmentes mit Membransegmenten,
- Figur 6A: ein Bild einer Mikrotiterplatte mit mehreren Wells, in welchen auf jeweiligen Trägerschichtsegmenten jeweilige Membransegmente positioniert sind,
- Figur 6B: ein vergrößertes Bild von Membransegmenten auf einem Trägerschichtsegment in einem Well,
- Figur 6C: Bildbereiche, welche jeweilige Membransegmente präsentieren,
- Figur 7A: Schritte einer ersten Ausführungsform eines Bildbearbeitungsverfahrens,
- Figur 7B: Schritte einer zweiten Ausführungsform eines digitalen Bildverarbeitungsverfahrens,
- Figur 8: Daten aus Experimentalergebnissen,
- Figur 9: einen Vergleich von Experimentalergebnissen.

Wie zuvor bereits erläutert, sind in den Figuren 2A bis 2E verschiedene Schritte des erfindungsgemäßen Verfahrens dargestellt.

Die Blotlinien BL1 verlaufen insbesondere parallel zueinander. Ferner verlaufen insbesondere die Blotlinien BL2 parallel zueinander.

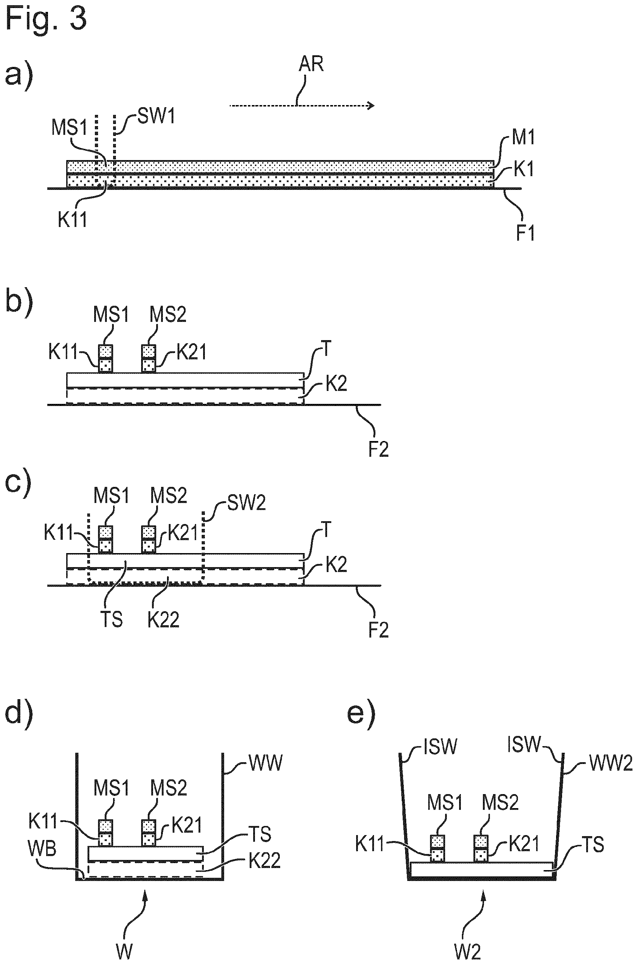

Figur 3A zeigt in einer Seitenansicht die erste Trägermembran M1, auf welcher entlang der Auftragsrichtung AR die Blotlinien aufgetragen sind. Diese Blotlinien sind in dieser Seitenansicht nicht separat dargestellt, können aber auf der Oberfläche der Membran M1 angenommen werden.

Es wird nun erläutert, auf welche Weise das Fixieren der Membransegmente auf der Trägerschicht vorzugsweise erfolgen kann. Ferner wird erläutert, auf welche Weise das Fixieren des Trägerschichtsegmentes in dem Well oberhalb des Wellbodens erfolgt.

Die Figur 3A zeigt in der Seitenansicht die Trägermembran M1, welche auf einer doppelseitigen Klebefolie K1 aufgebracht ist. Vorzugsweise befindet sich unterhalb der doppelseitigen Klebefolie K1 eine Schutzfolie F1.

Das Heraustrennen eines Membransegmentes MS1 erfolgt mittels Herausschneiden, bevorzugt mittels Ausstanzen. Besonders bevorzugt erfolgt das Ausstanzen mittels eines Stanzmessers.

Die doppelseitige Klebefolie K1 repräsentiert eine Klebeschicht. Die doppelseitige Klebefolie K1 weist insbesondere auf ihrer Ober- und ihrer Unterseite einen Haftvermittler auf, welcher je nach aufzubringendem Membranmaterial M1 gewählt werden kann. Derartige Haftvermittler sind beispielsweise aus der EP 2327636 B1 bekannt.

Die Klebeschicht K1 kann alternativ zu der Ausführungsform einer doppelseitigen Klebefolie vorzugsweise ein zuvor auf die Trägerschicht T aufgetragener UV-Kleber sein. Der UV-Kleber kann dann als Klebeschicht bezeichnet werden.

Das Heraustrennen des Membransegmentes MS1 erfolgt vorzugsweise mittels des Stanzwerkzeuges SW1. Das Heraustrennen des Membransegmentes MS1 erfolgt vorzugsweise in der Weise, dass ein Klebeschichtsegment K11 mit herausgetrennt wird. Das Membransegment MS1 kann dann, wie in der Figur 2D dargestellt, mittels des Klebeschichtsegmentes K11 auf einer Trägerschicht T fixiert werden.

Insbesondere kann bevorzugt mittels Einbringen von Druckluft in das Stanzwerkzeug SW1 die Kombination aus Membransegment MS1 und Klebeschichtsegment K11 aus dem Stanzwerkzeug SW1 ausgetrieben werden und so auf die Trägerschicht T aufgebracht werden.

Das Fixieren des Membransegmentes MS1 auf der Trägerschicht T erfolgt also mittels einer zwischen den Membransegmenten und der Trägerschicht befindlichen Klebeschicht bzw. eines Klebeschichtsegment K11.

Analoge Schritte können durchgeführt werden für eine zweite Trägermembran M2, so dass ein zweites Membransegment MS2 mittels einer Klebeschicht K21 auf der Trägerschicht TS fixiert wird.

Die Trägerschicht T befindet sich vorzugsweise auf einer weiteren Klebeschicht K2. Vorzugsweise befindet sich die weitere Klebeschicht K2 auf einer weiteren Schutzfolie F2.

Es erfolgt dann ein Heraustrennen des Trägerschichtsegmentes TS insbesondere mittels Herausschneiden, bevorzugt mittels Ausstanzen, besonders bevorzugt mittels eines Stanzmessers SW2 in Form eines Stanzwerkzeuges.

Bevorzugt erfolgt das Heraustrennen in der Weise, dass ein Klebeschichtsegment K22 bzw. eine Klebeschicht K22 mit herausgetrennt wird und dass dann das Fixieren des Trägerschichtsegmentes TS, wie in Figur 3D dargestellt, mittels der zwischen dem Wellboden WB und dem Trägerschichtsegment TS befindlichen Klebeschicht K22 erfolgt. Hierzu kann die Kombination aus Klebeschicht K22, Trägerschichtsegment TS, Klebeschichtsegmente K11, K21 und Membransegmente MS1, MS2 aus dem Stanzwerkzeug SW2 mittels Einbringen von Druck in das Stanzwerkzeug aus dem Werkzeug ausgetrieben und auf dem Wellboden positioniert und fixiert werden.

Eine weitere alternative Ausführungsform des erfindungsgemäßen Verfahrens wird nun unter Bezug auf Figur 3C dargelegt. In dem Fall, dass die Klebeschicht K2 nicht vorhanden ist, kann mittels eines Stanzwerkzeuges SW2 lediglich ein Trägerschichtsegment TS aus der Trägerschicht T herausgetrennt werden. Es kann dann, wie in der Figur 3E ersichtlich, das Fixieren des Trägerschichtsegmentes TS in dem Well W2, welches vorzugsweise konisch zulaufende Well-Wände WW2 aufweist, mittels Einklemmen des Trägerschichtsegmentes TS innerhalb der inneren Seitenwände ISW des Wells W2 erfolgen. Der Vorteil eines solchen Klemmverfahrens gemäß der Figur 3E ist, dass keine weitere Klebeschicht notwendig ist.

Der Vorteil eines Vorsehens einer weiteren Klebeschicht K22, wie in der Ausführungsform aus der Figur 3D dargestellt, ist, dass hierbei geringere Ansprüche an die Toleranzmaße der Grundfläche des Trägerschichtsegmentes TS gegenüber den Abmaßen des inneren Raumes des Wells W gestellt werden. Bei Verwendung einer Klebeschicht K22 sind möglicherweise auftretende Lücken zwischen dem Trägerschichtsegment TS und den Well-Wänden WW bei der Positionierung und Fixierung der Membransegmente tolerierbar. Dies ist nicht der Fall, falls das Trägerschichtsegment TS in dem Well W2 eingeklemmt wird, wie in Figur 3E dargestellt.

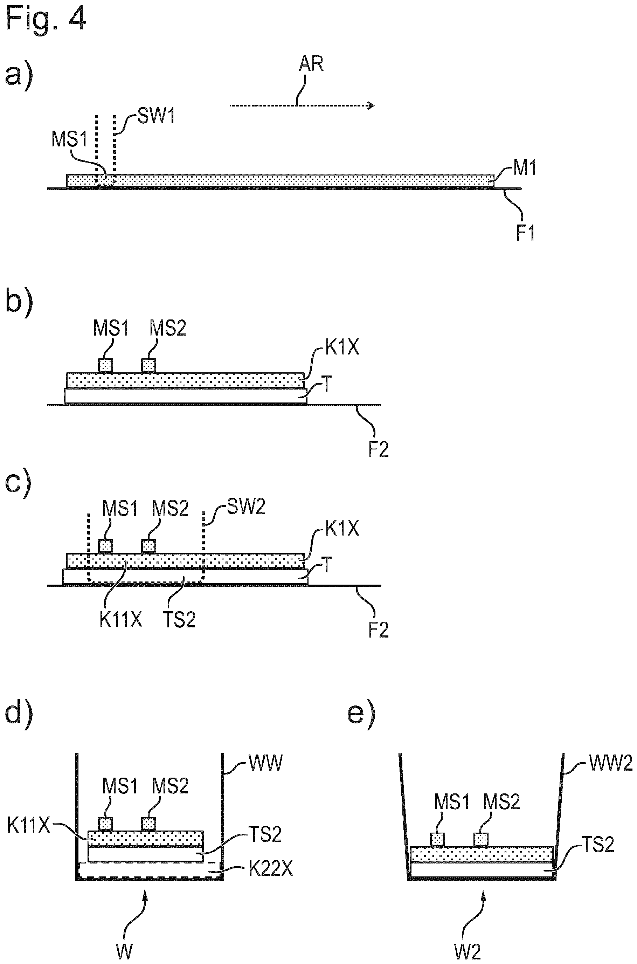

Figuren 4A-C zeigen eine weitere bevorzugte Ausführungsform des erfindungsgemäßen Verfahrens, bei welchem anstelle einer doppelseitigen Klebefolie als Klebeschicht alternativ ein flächig aufgetragener Kleber K1X verwendet wird.

Gemäß der Figur 4A wird in analoger Weise zu Figur 3A die Membran M1 mit in den Auftragungsrichtungen AR aufgetragenen Blotlinien bereitgestellt. Vorzugsweise ist eine Schutzfolie F1 vorhanden.

Es erfolgt dann ein Heraustrennen, besonders bevorzugt mittels eines Stanzwerkzeuges SW1, eines Membransegmentes MS1.

Die Figur 4B zeigt einen sich dann ergebenden Aufbau, bei welchem Membransegmente MS1, MS2 auf einer auf der Trägerschicht T flächig aufgetragenen Klebeschicht K1X positioniert und fixiert sind.

Die Figur 4C zeigt das Heraustrennen des Trägerschichtsegmentes TS2 aus der Trägerschicht T.

Gemäß der Figur 4D kann dann bevorzugt das Trägerschichtsegment TS2 mit den darauf befindlichen und positionierten und fixierten Membransegmenten MS1, MS2 in dem Well W mittels einer zuvor auf dem Wellboden aufgetragenen Klebeschicht K22X positioniert und fixiert werden.

Alternativ kann, wie in der Figur 4E dargestellt, das Trägerschichtsegment TS2 mittels Einklemmen des Trägerschichtsegmentes TS2 innerhalb der inneren Seitenwände des Wells W2 erfolgen.

Die Ausführungsformen aus der Figur 3D als auch der Figur 4D haben gemein, dass jeweils das Trägerschichtsegment TS, TS2 oberhalb des Wellbodens WB in dem Well W2 positioniert und fixiert ist, wobei ferner auf dem Trägerschichtsegment TS, TS2 zwei oder mehr positionierte und fixierte Membransegmente MS1, MS2 vorhanden sind, welche jeweils mehrere Blotliniensegmente aufweisen, wobei die jeweiligen Blotliniensegmente jeweilige Fängermolekültypen aufweisen. Ferner ist in diesen beiden Ausführungsformen aus der Figur 3D und 4D jeweils oberhalb des Trägerschichtsegmentes wenigstens eine Klebeschicht K11, K21, K11X vorhanden, auf welcher die Trägermembransegmente MS1, MS2 fixiert sind. Ferner ist in diesen Ausführungsformen aus der Figur 3D und 4D jeweils das Trägerschichtsegment TS, TS2 mittels einer weiteren Klebeschicht K22, K22X auf dem Wellboden WB des Wells W fixiert. Diese beiden Ausführungsformen bergen den Vorteil, dass eine genaue räumliche Positionierung der Membransegmente MS1, MS2 in dem Well bzw. oberhalb des Wellbodens WW dadurch gewährleistet werden kann, dass lediglich das Trägerschichtsegment TS, TS2 in Relation zu dem Wellboden innerhalb des Wells W2 mit einem Werkzeug sehr genau positioniert werden muss bei gegebenem geringen Bau bzw. Agitationsraum. Es kann hingegen die Positionierung der Membransegmente MS1, MS2 in Relation zu dem Trägerschichtsegment TS, TS2 außerhalb der Vertiefung des Wells erfolgen, wobei eben für diesen Schritt dann ein größerer Agitationsraum entsprechend der Werkzeuge gegeben ist als innerhalb des Wells.

Die Klebeschicht zur Fixierung der Trägermembransegmente MS1, MS2 aus dem Ausführungsbeispiel aus der Figur 3D ist durch mehrere Klebeschichtabschnitte K11, K21 gegeben.

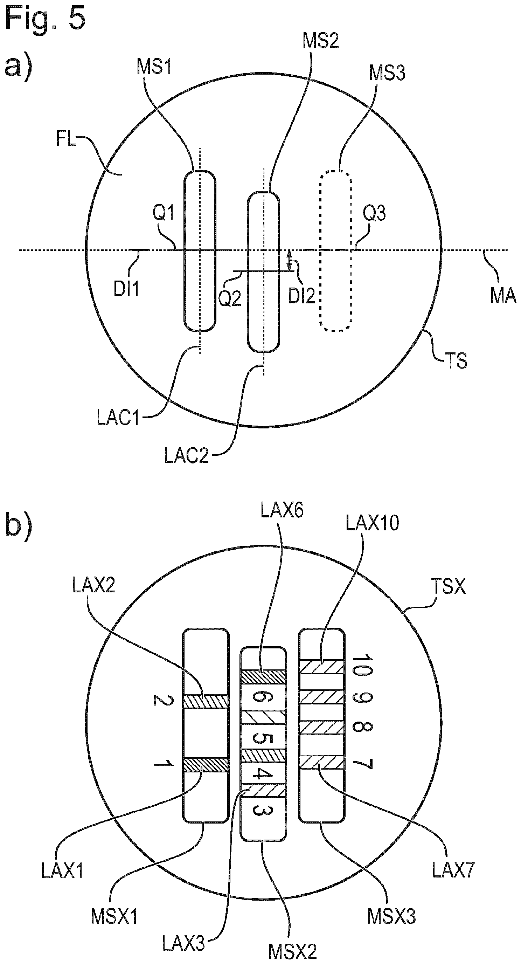

Die Figur 5A zeigt eine schematische Darstellung einer bevorzugten Ausführungsform eines Trägerschichtsegmentes TS mit wenigstens zwei Membransegmenten MS1, MS2. Bevorzugt ist ein weiteres, drittes Membransegment MS3 vorhanden.

Die Membransegmente MS1, MS2 weisen jeweils eine längliche Ausdehnung auf.

Ferner weisen die Membransegmente MS1, MS2 jeweils eine Längsachse LAC1, LAC2 entlang ihrer länglichen Ausdehnung auf. Ferner weisen die Membransegmente MS1, MS2 jeweils eine Querachse Q1, Q2 senkrecht zur Längsachse LAC1, LAC2 auf.

Das Trägerschichtsegment TS weist eine kreisförmige Grundfläche FL auf.

Das Positionieren der Membransegmente MS1, MS2 auf dem Trägerschichtsegment TS erfolgt in der Weise, dass die länglichen Membransegmente MS1, MS2 in besonderer Weise auf dem Trägerschichtsegment TS positioniert sind.

Die Trägermembransegmente MS1, MS2 sind mit ihrer jeweiligen Längsachse LAC1, LAC2 senkrecht zu einer bestimmten Mittelachse MA der kreisförmigen Grundfläche FL des Trägerschichtsegmentes TS positioniert.

Das erste Trägermembransegment MS1 ist mit seiner Querachse Q1 gegenüber der Mittelachse MA um eine erste Distanz DI1 versetzt positioniert. In diesem Ausführungsbeispiel beträgt diese Distanz DI1 Null (0).

Ferner ist das zweite Trägermembransegment MS2 mit seiner Querachse Q2 gegenüber der Mittelachse MA um eine zweite Distanz DI2 versetzt positioniert. Diese zweite Distanz DI2 unterscheidet sich von der ersten Distanz DI1.

Diese Ausführungsform eines Trägersegmentes TS mit Membransegmenten MS1, MS2 innerhalb einer Mikrotiterplatte ist daher besonders vorteilhaft, da aufgrund der räumlichen Anordnung der Membransegmente MS1, MS2 eindeutig eine Zuordnung entsprechender Bildbereiche MSX11, MSX12, siehe Figur 6C, der entsprechenden Membransegmente MS1, MS2 zu bestimmten Membransegmenttypen erfolgen kann.

Hierzu sei Figur 5B betrachtet. Beispielsweise kann das erste Membransegment MS1 aus Figur 5A ein Membransegment MSX1 sein, auf welchem ein erstes Liniensegment LAX1 einer Blotlinie vorhanden ist. Ferner kann beispielsweise ein zweites Liniensegment LAX2 vorhanden sein. Das Liniensegment LAX1 kann eine Serumkontrolle sein. Das Liniensegment LAX2 kann eine Konjugatkontrolle sein.

Das zweite Membransegment MS2 kann ein Membransegment MSX2 sein, welches vier verschiedene Banden bzw. Liniensegmente von Blotlinien aufweist. Dieses können Liniensegmente LAX3 bis LAX6 sein. Beispielsweise kann das Liniensegment LAX3 einen ersten Fängermolekültyp aufweisen sowie das Liniensegment LAX4 einen zweiten Fängermolekültyp.

Für eine automatisierte Bildauswertung ist es beispielsweise notwendig, eine 360°-Eindeutigkeit eines aufgenommenen Bildes eines Trägersegmentes mit Membransegmenten darauf feststellen zu können, um beispielsweise eine Vertauschung des Membransegmentes MSX1 mit dem Membransegment MSX2 zu vermeiden.

Aufgrund der besonderen Lage der Querachsen Q1, Q2 der Membransegmente MS1, MS2, siehe Figur 5A, gegenüber der Mittelachse MA kann dann also eine klare Identifikation bestimmter Bildbereiche der bestimmten Membransegmente MS1, MS2 gewährleistet werden. Es kann insbesondere bei Bereitstellen eines Datensatzes, welcher zu bestimmten Membransegmenttypen MSX1,MSX2 bestimmte Abfolgen von Liniensegmenten LAX1, LAX2 sowie LAX3 bis LAX6 bzw. entsprechender Fängermolekültypen indiziert, dann jeweils eine automatisierte Auswertung eines entsprechenden Bildes hinsichtlich der bestimmten Fängermolekültypen ermöglicht werden.

Es kann also eine hier vorgeschlagene Mikrotiterplatte aufweisend entsprechende Trägermembransegmente mit einer Patientenprobe, einem Konjugat und einem Substrat inkubiert werden. Es kann dann ferner ein Bild erfasst werden, welches das Trägerschichtsegment und die Trägermembransegmente repräsentiert. Die Figur 6A zeigt hierzu ein Ausführungsbeispiel einer Mikrotiterplatte MTX mit entsprechenden Membransegmenten und darauf befindlichen erscheinenden Verfärbungen auf den Banden bzw. Liniensegmenten.

Die Figur 6B zeigt ein beispielhaftes Bild B, welches ein Trägerschichtsegment mit darauf befindlichen Trägermembransegmenten repräsentiert.

Die Figur 6C zeigt jeweilige Bildausschnitte des Bildes B aus der Figur 6B, welche jeweilige Membransegmente MSX11, MSX12, MSX13 repräsentieren.

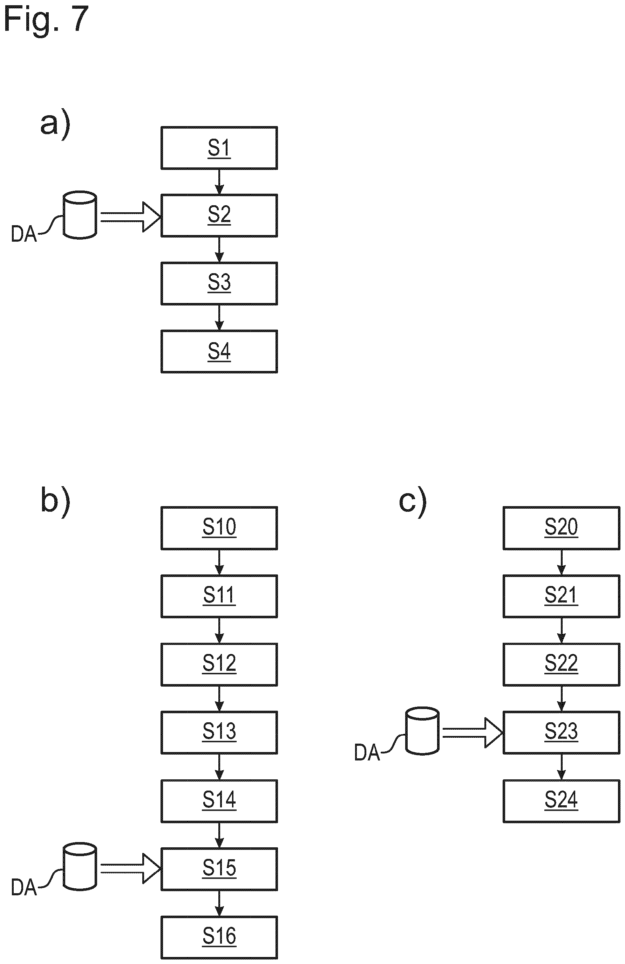

Die Figur 7A zeigt prinzipielle Schritte eines digitalen Bildverarbeitungsverfahrens.

In einem Schritt S1 erfolgt ein Erfassen des Bildes, welches das Trägerschichtsegment und die Trägermembransegmente repräsentiert.

In einem Schritt S2 erfolgt ein Identifizieren des ersten Trägermembransegmentes und des zweiten Trägermembransegmentes in dem Bild, insbesondere mittels eines bereitgestellten Datensatzes DA. Dieser Datensatz indiziert wenigstens zwei Typen von Trägermembransegmenten sowie jeweilige Lagen der Typen der Trägermembransegmente gegenüber einer Mittelachse einer kreisförmigen Fläche eines Trägerschichtsegmentes. Alternativ indiziert der Datensatz DA Distanzen von Querachsen der Membransegmente gegenüber einer Mittelachse der kreisförmigen Fläche des Trägerschichtsegmentes.

In einem Schritt S3 erfolgt ein Bestimmen jeweiliger Bindungsmaße, welche eine jeweilige Bindung von Antikörpern oder Antigenen an jeweilige Blotliniensegmente indizieren. Derartige Bindungsmaße können aus Grauwerten entsprechender Banden bzw. Liniensegmente oder Linienabschnitten der Membransegmente abgeleitet werden.

In einem Schritt S4 erfolgt ein Ausgeben der jeweiligen Bindungsmaße.

Die Figur 7B zeigt eine erste besondere Ausführungsform des in Bezug auf Figur 7A erläuterten Verfahrens.

Gemäß der Figur 7B erfolgt in einem Schritt S10 die Aufnahme eines Bildes. In einem Schritt S11 wird in dem Bild eine kreisförmige Fläche des Trägerschichtsegmentes, wie in Figur 5A dargestellt, mittels eines Segmentierungsschrittes erkannt. In einem Schritt S12 erfolgt ein Erkennen jeweiliger Membransegmentflächen mittels einer Segmentierung. In einem Schritt S13 erfolgt ein Bestimmen der Querachsen der Membransegmentflächen. Ferner erfolgt in einem Schritt S14 ein Bestimmen der Mittelachse des kreisförmigen Trägerschichtsegmentes, wobei diese Mittelachse parallel zu den Querachsen der Membransegmentflächen verläuft.

Es erfolgt dann in einem Schritt S15 ein Bestimmen der Distanzen der Querachsen zu der Mittelachse. Ferner wird ein Datensatz DA bereitgestellt, welcher vorzugsweise als höheres Wissen bezeichnet werden kann. Dieser Datensatz DA indiziert für jeweilige Distanzen jeweiliger Querachsen gegenüber der Mittelachse jeweils einen jeweiligen Membransegmenttyp. Insbesondere indiziert der Datensatz für jeden Membransegmenttyp eine entsprechende räumliche Abfolge von Liniensegmenten bzw. entsprechender Banden mit Fängermolekültypen auf dem entsprechenden Membransegment. Hieraus kann dann die Abfolge der Banden bzw. Liniensegmente mit dem entsprechenden Fängermolekül in einem entsprechenden Teilbild eines Membransegmentes, wie in Figur 6C dargestellt, abgeleitet werden.

In einem Schritt S16 erfolgt eine Analyse der Bandenintensität bzw. der Liniensegmente mittels einer digitalen Bildverarbeitung einer Ausgabe entsprechender Bindungsmaße, welche jeweilige Bindung von Antikörpern oder Antigenen an jeweilige Blotliniensegmente indizieren.

Ein solches Bindungsmaß kann beispielsweise ein mittlerer Graubereich mit einer Bande bzw. eines Liniensegmentes sein. Ferner kann beispielsweise ein solches Bindungsmaß ein qualitatives Bindungsmaß sein, welches eine positive bzw. negative Entscheidung indiziert.

Die Figur 7C zeigt ein alternatives Bildverarbeitungsverfahren gegenüber dem der Figur 7B.

Gemäß der Figur 7C erfolgt eine Aufnahme des Bildes in dem Schritt S20. Ferner erfolgt mittels Segmentierung ein Erkennen der Membransegmentflächen in einem Schritt S21. Ferner erfolgt in einem Schritt S22 ein Bestimmen der Querachsen der Membransegmentflächen. In dem Fall, dass das Trägersegment TS, siehe Ausführungsbeispiel aus der Figur 5A, drei Membransegmente MS1, MS2, MS3 aufweist, können dann die jeweiligen Lagen der jeweiligen Querachsen bestimmt werden. Ferner können für jede Querachse jeweils die Relationsdistanzen in Längsrichtung bzw. orthogonal zur Querachse zu den jeweils anderen Querachsen bestimmt werden. Somit wird für eine bestimmte Querachse dann eine minimale Distanz gegenüber einer anderen, am nächsten befindlichen Querachse bestimmt. In diesem Ausführungsbeispiel aus Figur 5A weist beispielsweise die Querachse Q1 eine minimale Distanz DI1 von 0 (Null) gegenüber der Querachse Q3 auf. Die Querachse Q2 weist eine minimale Distanz DI2 gegenüber der Querachse Q1 als auch der Querachse Q3 auf. Somit kann die Querachse Q2 und damit das Membransegment MS2 eindeutig gegenüber den Membransegmenten MS1 und MS3 identifiziert werden. Es kann dann also als höheres Wissen in Form eines Datensatzes DA einfließen, dass ein bestimmtes Membransegment, hier das Membransegment MS2, mit seiner Querachse eine größte minimale Distanz zu allen anderen Querachsen aufweist. Somit können dann die jeweiligen Membransegmenttypen bzw. Membransegmente MS1, MS2, MS3 bzw. MSX1, MSX2, MSX3 identifiziert werden, um auch ihre räumliche Orientierung und so die Abfolge der entsprechenden Liniensegmente LAX1, LAX2, LAX3, LAX6, LAX7, LAX10 sicher zu identifizieren.

Es ergibt sich also daraus ein Ableiten der Abfolge, welche Arten von Banden bzw. welche Arten von Fängermolekültypen auf den einzelnen Membransegmenten zu erwarten sind.

In einem Schritt S24 erfolgt dann die Analyse der Bandenintensitäten via Bildverarbeitung und die Ausgabe der jeweiligen Bindungsmaße für die jeweiligen Banden.

In beiden Ausführungsformen aus Figur 7B und Figur 7C indiziert der Datensatzes für jeweilige räumliche Lagen jeweiliger Querachsen jeweilige Membransegmenttypen. Insbesondere indiziert der Datensatz für jeden Membransegmenttyp und eine entsprechende räumliche Abfolge von Liniensegmenten bzw. entsprechender Banden mit Fängermolekültypen auf dem entsprechenden Membransegment. Der Datensatz kann auch in Form von mehreren Teildatensätzen gegeben sein.

Offenbart ist also auch ein Verfahren umfassend verschiedene Schritte. In einem Schritt wird eine Mikrotiterplatte bereitgestellt, für welche die Trägermembransegmente MS1, MS2 jeweils eine längliche Ausdehnung, ferner eine Längsachse LAC1, LAC2 entlang der länglichen Ausdehnung sowie ferner eine Querachse Q1, Q2 senkrecht zur Längsachse LAC1, LAC2 aufweisen, wobei ferner das Trägerschichtsegment TS eine kreisförmige Grundfläche FL aufweist, und wobei ferner die länglichen Trägermembransegmente MS1, MS2 in der Weise auf dem Trägerschichtsegment TS positioniert sind, dass
- die Trägermembransegmente MS1, MS2 mit ihrer jeweiligen Längsachse LAC1, LAC2 jeweils senkrecht zu einer Mittelachse MA der kreisförmigen Grundfläche FL des Trägerschichtsegmentes TS positioniert sind,
- dass ferner das erste Trägermembransegment MS1 mit seiner Querachse Q1 zu der Mittelachse MA um eine erste Distanz DI1 versetzt positioniert ist
- und dass ferner das zweite Trägermembransegment MS2 mit seiner Querachse Q2 zu der Mittelachse MA um eine zweite Distanz DI2 versetzt positioniert ist, welche sich von der ersten Distanz DI1 unterscheidet.

In einem weiteren Schritt erfolgt ein Erfassen eines Bildes B, welches das Trägerschichtsegment TS und die Trägermembransegmente MS1, MS2 repräsentiert. In einem weiteren Schritt erfolgt ein Bereitstellen eines Datensatzes, welcher für jeweilige räumliche Lagen jeweiliger Querachsen jeweilige Membransegmenttypen indiziert. In einem weiteren Schritt erfolgt ein Identifizieren des ersten Trägermembransegmentes MS1 und des zweiten Trägermembransegmentes MS2 in dem Bild B unter Verwendung des Datensatzes. In einem weiteren bevorzugten Schritt erfolgt ein Bereitstellen von Teilbildern MSX11, MSX12, welche die jeweiligen Membransegmente MS1, MS2 repräsentieren sowie ein Indizieren der jeweiligen Membransegmenttypen zu den jeweiligen Teilbildern MSX11, MSX12. Dieses Indizieren der Membransegmenttypen kann dadurch erfolgen, dass die Teilbilder MSX11, MSX12 in einer bestimmten Reihenfolge bereitgestellt werden. Alternativ kann dieses Indizieren dadurch erfolgen, dass ein jeweiliges Teilbild MSX11, MSX12 zusammen mit einem jeweiligen Teildatensatz bereitgestellt wird, welcher den jeweiligen Membransegmenttyp für ein jeweiliges Teilbild indiziert.

Ferner erfolgt vorzugsweise ein Bestimmen jeweiliger Bindungsmaße, welche eine jeweilige Bindung von Antikörpern oder Antigenen an jeweilige Blotliniensegmente indizieren sowie ein Ausgeben der jeweiligen Bindungsmaße.

Im Sinne dieser Anmeldung können die Membransegmente vorzugsweise als Blotstreifen bzw. Linienblotstreifen bezeichnet werden Referenz 1 (Raoult, D, and Dasch, G. A., 1989, The line blot: an immunoassay for monoclonal and other antibodies. Its application to the serotyping of gram-negative bacteria. J. Immunol. Methods, 125 (1-2), 57-65; WO2013041540). Vorzugsweise wird der Begriff "Blotstreifen" bzw. "Linienblot" oder auch "Membransegment" im Sinne dieser Anmeldung als ein Teststreifen angesehen, besonders bevorzugt als ein membranbasierter Streifen, welcher mit wenigstens zwei verschiedenen Stoffen zum Einfangen von Antikörpern oder Antigenen beschichtet ist, wobei die Stoffe vorzugsweise Polypeptide sind. Diese zwei Stoffe sind vorzugsweise als jeweilige Liniensegmente räumlich getrennt voneinander auf dem Membransegment bzw. dem Linienblot bzw. dem Teststreifen aufgebracht.

Ein solches Membransegment bzw. ein solcher Teststreifen oder auch Linienblot weist vorzugsweise einen oder mehrere Bereiche auf, welche eine Kontrollbande genannt werden, wobei eine solche Kontrollbande geeignet ist zum Bestätigen, dass das Membransegment bzw. Linienblot bzw. Teststreifen lang genug und bei angemessenen Bedingungen mit einer Patientenprobe kontaktiert wurde. Die Patientenprobe ist vorzugsweise menschliches Blut, bevorzugt menschliches Blutplasma oder menschliches Blutserum, besonders bevorzugt verdünntes menschliches Blutplasma oder verdünntes menschliches Blutserum. Unterschiedliche Linienblots zweier Blotstreifen sind beschrieben im Stand der Technik, beispielsweise in Referenz 2 (van Oss, J. C., und Regenmortel, M. H. V., Immunochemistry, 1994, Marcell Dekker, insbesondere Kapitel 35).

Im Sinne dieser Anmeldung ist eine Trägermembran vorzugsweise eine Nitrozellulosemembran, eine Nylonmembran oder eine PVDF-Membran. Die Trägermembran ist somit geeignet, eine Lösung aus Fängermolekül und Puffer zu adsorbieren. Insbesondere erfolgt das Beschichten der Membran mit einem Fängermolekül, indem eben eine solche Lösung auf die Membran aufgetragen wird.

Im Sinne dieser Anmeldung ist eine Trägerschicht vorzugsweise eine mechanisch feste Kunststoffschicht oder eine mechanisch flexible Kunststofffolie. Der Kunststoff kann insbesondere PET (Polyethylenterephthalat) sein, insbesondere antistatischer PET.

Im Sinne dieser Anmeldung ist ein Blockierungs-Buffer ein Puffer, der eine unspezifische Bindung des Nachweisantikörpers oder des Nachweisantigens an die Oberfläche der Membran verhindert. Ein solcher Puffer enthält entweder Proteine oder andere Komponenten zum Blockieren von Bindungsstellen, so dass unspezifische Bindungen verhindert werden.

Im Sinne dieser Anmeldung kann eine doppelseitige Klebefolie eine Folie sein, welche auf beiden Seiten mit einem zuvor dargelegten Haftvermittler beschichtet ist.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, und nicht in einem idealisierten oder übermäßig formalen Sinn zu interpretieren sind, solange dies hierin nicht ausdrücklich definiert ist.

Die in der vorstehenden Beschreibung, den Ansprüchen und den Zeichnungen offenbarten Merkmale können sowohl einzeln als auch in beliebiger Kombination für die Verwirklichung von Ausführungsbeispielen in ihren verschiedenen Ausgestaltungen von Bedeutung sein und - soweit sich nicht aus der Beschreibung etwas anderes ergibt - beliebig miteinander kombiniert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Merkmals einer entsprechenden Vorrichtung dar.

### ERGEBNISSE

Es wurde eine Beschichtung von drei Membranen mit einer Auswahl unterschiedlicher Antigene und auch Antikörper entsprechend dem EUROIMMUN Produkt Anti-Borrelia-EUROLINE-RN-AT (IgG) vorgenommen und entsprechende Membransegmente auf einem Trägerschichtsegment in Wells einer Mikrotiterplatte des Herstellers Biomat eingebracht und inkubiert. Parallel wurden Lineblotstreifen mit entsprechenden Antigenen und auch Antikörpern hergestellt und ebenfalls inkubiert.

Die Abarbeitung der Lineblotstreifen erfolgte in einer Blotwanne manuell nach Anleitung DN_2131G_A_DE_C11 des Produktes Anti-Borrelia-EUROLINE-RN-AT (IgG). Die Abarbeitung der Antigene und der Antikörper in der Mikrotiterplatte erfolgte nach der gleichen Anleitung unter Anpassung der Volumina der Reagenzien. Bei dieser Anpassung wurde der Volumenunterschied zwischen der Blotwanne und dem Well der Mikrotiterplatte berücksichtigt.

Die Bildaufnahme und Auswertung der Lineblotstreifen erfolgte in einem ersten Ansatz mit den Produkten EUROBlotScanner und EUROLineScan. Die Bildaufnahme der Wells erfolgte in einem zweiten Ansatz mit einem neu entwickelten Bildaufnahmesystem, die Auswertung der Wellbilder erfolgte ebenfalls mit dem Produkt EUROLineScan.

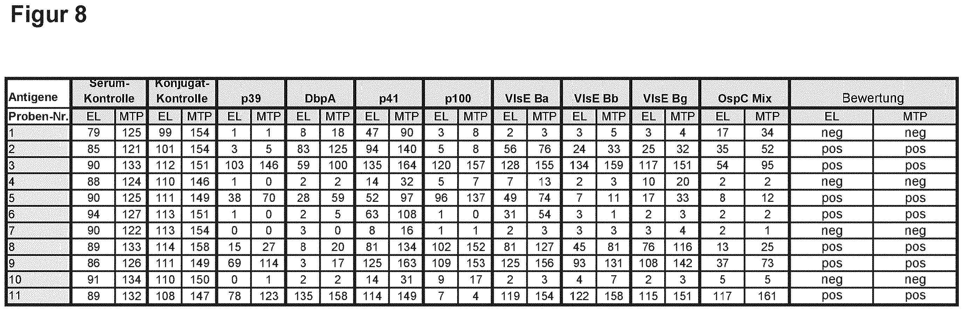

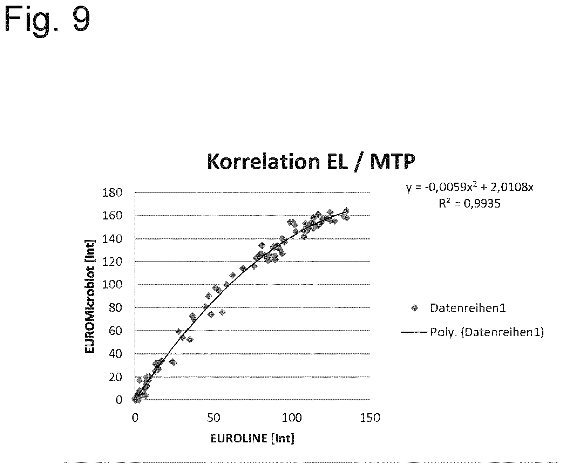

Die Figur 8 zeigt eine Tabelle mit Ergebnissen. Für unterschiedliche Antigene als auch Antikörper und unterschiedliche Nummern von Patientenproben findet sich jeweils ein Intensitätswert der entsprechenden Bande für einen Lineblotstreifen (EL) sowie für ein Well einer Mikrotiterplatte (MTP). Die Intensitätswerte der beiden Ansätze sind vergleichbar bzw. korrelieren stark miteinander, wie später auch unter Bezug auf Figur 9 noch dargelegt. Die jeweiligen positiv/negativ Bewertungen für die jeweiligen Ansätze Lineblotstreifen (EL) bzw. Mikrotiterplatte (MTP) unter Anwendung jeweiliger, individueller Schwellenwerte für die jeweiligen Ansätze finden sich jeweils in den letzten beiden Spalten. Hier ist eine Übereinstimmung aller jeweiligen positiv/negativ Bewertungen festzustellen.

Bei der Serumkontrolle und der Konjugatkontrolle handelte es sich jeweils um Antikörper als Fängermoleküle. Diese Antikörper der jeweiligen Kontrollen sind jeweils Antihuman-Antikörper. Das Antigen mit der Bezeichnung p39 ist auch unter der Bezeichnung BmpA bekannt, eine entsprechende Uniprot-Nummer lautet P0C223. Das Antigen mit der Bezeichnung DbpA hat eine entsprechende Uniprot-Nummer O50917. Das Antigen mit der Bezeichnung p41 ist auch unter der Bezeichnung Flagellin bekannt, eine entsprechende Uniprot-Nummer lautet C3VMP5. Das Antigen mit der Bezeichnung p100 hat eine entsprechende Uniprot-Nummer Q45013. Das Antigen mit der Bezeichnung VIsE Ba ist auch unter der Bezeichnung VIsE Borrelia afzelii bekannt, eine entsprechende Uniprot-Nummer lautet Q5DVM0. Das Antigen mit der Bezeichnung VIsE Bb ist auch unter der Bezeichnung VIsE Borrelia burgdorferi bekannt, eine entsprechende Uniprot-Nummer lautet O06878. Das Antigen mit der Bezeichnung VIsE Bg ist auch unter der Bezeichnung VIsE Borrelia garinii bekannt, eine entsprechende Uniprot-Nummer lautet Q5DVM1. Das Antigen mit der Bezeichnung OspC Mix ist auch unter der Bezeichnung OspC bekannt, eine entsprechende Uniprot-Nummer lautet Q07337.

Figur 9 zeigt eine Korrelation der Intensitätswerte für übliche Lineblotstreifen (EUROLINE) auf der x-Achse sowie Wells von Mikrotiterplatten (EUROMicroblot) auf der y-Achse über alle Antigene als auch Antikörper und alle Patientenproben. Figur 9 zeigt ferner eine Fitting Curve in Form einer quadratischen Funktion mittels Regressionsverfahren. Das Bestimmtheitsmaß nimmt hier einen Wert von R² = 0,9935 an.

## Patentansprüche

1. Verfahren zum Herstellen einer Mikrotiterplatte (MTX) mit
Fängermolekülbeschichtungen,
wobei die Fängermoleküle Antigene oder Antikörper sind,
umfassend
- Bereitstellen einer ersten Trägermembran (M1) sowie einer zweiten Trägermembran (M2),
- Beschichten der ersten Trägermembran (M1) mit jeweiligen ersten Blotlinien (BL1), welche jeweils einen jeweiligen Fängermolekültypen aufweisen,
- Beschichten der zweiten Trägermembran (M2) mit jeweiligen zweiten Blotlinien (BL2), welche jeweils einen jeweiligen Fängermolekültypen aufweisen,
- Heraustrennen eines ersten Membransegmentes (MS1) aus der ersten Trägermembran (M1), welches jeweilige Linienabschnitte (LA11, ..., LA14) der jeweiligen ersten Blotlinien (BL1) aufweist,
- Heraustrennen eines zweiten Membransegmentes (MS2) aus der zweiten Trägermembran (M2), welches jeweilige Linienabschnitte (LA21, ..., LA23) der jeweiligen zweiten Blotlinien (BL2) aufweist,
- Positionieren und Fixieren des ersten Membransegmentes (MS1) auf einer Trägerschicht (T),
- Positionieren und Fixieren des zweiten Membransegmentes (MS2) auf der Trägerschicht (T),
- Heraustrennen eines Trägerschichtsegmentes (TS) aus der Trägerschicht (T), welches das erste und das zweite Membransegment (MS1, MS2) aufweist,
- Positionieren und Fixieren des Trägerschichtsegmentes (TS) in einem Well (W, W2) der Mikrotiterplatte (MTX).

2. Verfahren nach Anspruch 1,
wobei die ersten Blotlinien (BL1) parallel zueinander verlaufende Blotlinien sind, und wobei die zweiten Blotlinien (BL2) parallel zueinander verlaufende Blotlinien sind.

3. Verfahren nach Anspruch 1,
ferner umfassend
- Blockieren der ersten Blotlinien (BL1) auf der ersten Trägermembran (M1) mit einem ersten Blocking Buffer
- sowie Blockieren der zweiten Blotlinien (BL2) auf der zweiten Trägermembran (M2) mit einem zweiten Blocking Buffer.

4. Verfahren nach Anspruch 1,
wobei das Heraustrennen der Membransegmente (MS1, MS2) mittels Herausschneiden erfolgt, bevorzugt mittels Ausstanzen.

5. Verfahren nach Anspruch 1,
wobei die Membransegmente (MS1, MS2) jeweils
- eine längliche Ausdehnung,
- ferner eine Längsachse (LAC1, LAC2) entlang der länglichen Ausdehnung
- sowie ferner eine Querachse (Q1, Q2) senkrecht zur Längsachse (LAC1, LAC2) aufweisen.

6. Verfahren nach Anspruch 5,
wobei das Trägerschichtsegment (TS) eine kreisförmige Grundfläche (FL) aufweist, wobei ferner das Positionieren der Membransegmente (MS1, MS2) und das Heraustrennen des Trägerschichtsegmentes (TS) so erfolgt, dass die länglichen Membransegmente (MS1, MS2) in der Weise auf dem Trägerschichtsegment (TS) positioniert sind, dass
- die Membransegmente (MS1, MS2) mit ihrer jeweiligen Längsachse (LAC1, LAC2) jeweils senkrecht zu einer Mittelachse (MA) der kreisförmigen Grundfläche (FL) des Trägerschichtsegmentes (TS) positioniert sind,
- ferner das erste Membransegment (MS1) mit seiner Querachse (Q1) zu der Mittelachse (MA) um eine erste Distanz (DI1) versetzt positioniert ist
- und ferner das zweite Membransegment (MS2) mit seiner Querachse (Q2) zu der Mittelachse (MA) um eine zweite Distanz (DI2) versetzt positioniert ist, welche sich von der ersten Distanz (DI1) unterscheidet.

7. Verfahren nach Anspruch 1,
wobei das Fixieren der Membransegmente (MS1, MS2) auf der Trägerschicht (T) mittels einer zwischen den Membransegmenten (MS1, MS2) und der Trägerschicht (T) befindlichen Klebeschicht (K11, K21, K1X, K11X) erfolgt.

8. Verfahren nach Anspruch 1,
wobei das Fixieren des Trägerschichtsegmentes (TS) in dem Well (W) auf dem Boden (WB) des Wells (W) mittels einer zwischen dem Wellboden (WB) und dem Trägerschichtsegment (TS) befindlichen Klebeschicht (K22, K22X) erfolgt.

9. Verfahren nach Anspruch 1,
wobei das Fixieren des Trägerschichtsegmentes (TS) in dem Well (W2) mittels Einklemmen des Trägerschichtsegmentes (TS) innerhalb der inneren Seitenwand (IS) des Wells (WS) erfolgt.

10. Mikrotiterplatte (MTX) mit wenigstens einem Well (W, W2) mit Fängermolekülbeschichtungen,
wobei die Fängermoleküle Antigene oder Antikörper sind,
aufweisend
- ein Trägerschichtsegment (TS), welches oberhalb eines Wellbodens in dem Well (W, W2) positioniert und fixiert ist,
- ferner auf dem Trägerschichtsegment (TS) zwei oder mehr positionierte und fixierte Trägermembransegmente (MS1, MS2), welche jeweils mehrere Blotliniensegmente (LA11, ..., LA14, LA21, ..., LA23) aufweisen, wobei die jeweiligen Blotliniensegmente jeweilige Fängermolekültypen aufweisen.

11. Mikrotiterplatte nach Anspruch 10,
wobei die Blotliniensegmente eines Trägermembransegmentes (MS1, MS2) parallel zueinander verlaufende Blotliniensegmente sind.

12. Mikrotiterplatte nach Anspruch 11,
ferner umfassend oberhalb des Trägerschichtsegmentes (TS) wenigstens eine Klebeschicht (K11, K22, K1X, K11X), auf welcher die Trägermembransegmente (MS1, MS2) fixiert sind.

13. Mikrotiterplatte nach Anspruch 12,
wobei die Klebeschicht aus mehreren Klebeschichtabschnitten (K11, K21) besteht und wobei die Klebeschichtabschnitte (K11, K21) jeweils Abschnitte einer doppelseitigen Klebefolie (K1) sind.

14. Mikrotiterplatte nach Anspruch 12,
wobei ferner das Trägerschichtsegment (TS) mittels einer weiteren Klebeschicht (K22, K2X) auf dem Wellboden (WB) des Wells (W) fixiert ist,
wobei ferner die weitere Klebeschicht (K22) vorzugsweise eine doppelseitige Klebefolie (K2) ist.

15. Mikrotiterplatte nach Anspruch 10,
wobei die Trägermembransegmente (MS1, MS2) jeweils
- eine längliche Ausdehnung,
- ferner eine Längsachse (LAC1, LAC2) entlang der länglichen Ausdehnung
- sowie ferner eine Querachse (Q1, Q2) senkrecht zur Längsachse (LAC1, LAC2) aufweisen,
wobei ferner das Trägerschichtsegment (TS) eine kreisförmige Grundfläche (FL) aufweist,
und wobei ferner die länglichen Trägermembransegmente (MS1, MS2) in der Weise auf dem Trägerschichtsegment (TS) positioniert sind, dass
- die Trägermembransegmente (MS1, MS2) mit ihrer jeweiligen Längsachse (LAC1, LAC2) jeweils senkrecht zu einer Mittelachse (MA) der kreisförmigen Grundfläche (FL) des Trägerschichtsegmentes (TS) positioniert sind,
- ferner das erste Trägermembransegment (MS1) mit seiner Querachse (Q1) zu der Mittelachse (MA) um eine erste Distanz (DI1) versetzt positioniert ist
- und ferner das zweite Trägermembransegment (MS2) mit seiner Querachse (Q2) zu der Mittelachse (MA) um eine zweite Distanz (DI2) versetzt positioniert ist, welche sich von der ersten Distanz (DI1) unterscheidet.

16. Verfahren
umfassend
- Bereitstellen einer Mikrotiterplatte (MTX) nach einem der Ansprüche 10 bis 15 aufweisend die Trägermembransegmente (MS1, MS2),
- Inkubieren der Trägermembransegmente (MS1, MS2) mit einer Patientenprobe, einem Konjugat und einem Substrat.

17. Verfahren nach Anspruch 16,
ferner umfassend
- Erfassen eines Bildes (B), welches das Trägerschichtsegment (TS) und die Trägermembransegmente (MS1, MS2) repräsentiert,
- Identifizieren des ersten Trägermembransegmentes (MS1) und des zweiten Trägermembransegmentes (MS2) in dem Bild (B),
- Bestimmen jeweiliger Bindungsmaße, welche eine jeweilige Bindung von Antikörpern oder Antigenen an jeweilige Blotliniensegmente indizieren,
- Ausgeben der jeweiligen Bindungsmaße.

18. Verfahren nach Anspruch 17,
wobei die Mikrotiterplatte eine Mikrotiterplatte nach Anspruch 15 ist,
ferner umfassend
- Bereitstellen eines Datensatzes (DA), welcher für jeweilige räumliche Lagen jeweiliger Querachsen jeweilige Membransegmenttypen indiziert,
- Identifizieren des ersten Trägermembransegmentes (MS1) und des zweiten Trägermembransegmentes (MS2) in dem Bild (B) unter Verwendung des Datensatzes,
- Bereitstellen von Teilbildern (MSX11, MSX12), welche die jeweiligen Membransegmente (MS1, MS2) repräsentieren.

## Claims

1. Method for producing a microtitration plate (MTX) having capture molecular coatings, wherein the capture molecules are antigens or antibodies,
comprising the following steps:
- providing a first carrier membrane (M1) and a second carrier membrane (M2);
- coating the first carrier membrane (M1) with the respective first blot lines (BL1) which each comprise a respective capture molecule type;
- coating the second carrier membrane (M2) with the respective second blot lines (BL2) which each comprise a respective capture molecule type;
- removing from the first carrier membrane (M1) a first membrane segment (MS1) which comprises respective line portions (LA11, ..., LA14) of the respective first blot lines (BL1);
- removing from the second carrier membrane (M2) a second membrane segment (MS2) which comprises respective line portions (LA21, ..., LA23) of the respective second blot lines (BL2);
- positioning and fixing the first membrane segment (MS1) on a carrier layer (T);
- positioning and fixing the second membrane segment (MS2) on the carrier layer (T);
- removing from the carrier layer (T) a carrier layer segment (TS) which comprises the first and the second membrane segment (MS1, MS2);
- positioning and fixing the carrier layer segment (TS) in a well (W, W2) of the microtitration plate (MTX).

2. Method according to Claim 1,
wherein the first blot lines (BL1) are blot lines which run so as to be mutually parallel, and wherein the second blot lines (BL2) are blot lines which run so as to be mutually parallel.

3. Method according to Claim 1,
furthermore comprising the following steps:
- blocking the first blot lines (BL1) on the first carrier membrane (M1) with a first blocking buffer; and
- blocking the second blot lines (BL2) on the second carrier membrane (M2) with a second blocking buffer.

4. Method according to Claim 1,
wherein the removal of the membrane segments (MS1, MS2) is performed by means of cutting, preferably by means of punching.

5. Method according to Claim 1,
wherein the membrane segments (MS1, MS2) each comprise
- an elongate extent;
- furthermore a longitudinal axis (LAC1, LAC2) along the elongate extent; and
- furthermore a transverse axis (Q1, Q2) which is perpendicular to the longitudinal axis (LAC1, LAC2).

6. Method according to Claim 5,
wherein the carrier layer segment (TS) has a circular base area (FL), wherein furthermore the positioning of the membrane segments (MS1, MS2) and the removal of the carrier layer segments (TS) is performed in such a way that the elongate membrane segments (MS1, MS2) are positioned on the carrier layer segments (TS) in such a manner that
- the membrane segments (MS1, MS2) by way of their respective longitudinal axis (LAC1, LAC2) are in each case positioned perpendicularly to a central axis (MA) of the circular base area (FL) of the carrier layer segment (TS);
- furthermore the first membrane segment (MS1) by way of its transverse axis (Q1) is positioned so as to be offset from the central axis (MA) by a first distance (DI1); and
- furthermore the second membrane segment (MS2) by way of its transverse axis (Q2) is positioned so as to be offset from the central axis (MA) by a second distance (DI2) which is different from the first distance (DI1).

7. Method according to Claim 1,
wherein the fixing of the membrane segments (MS1, MS2) on the carrier layer (T) is performed by means of an adhesive layer (K11, K21, K1X, K11X) which is located between the membrane segments (MS1, MS2) and the carrier layer (T).

8. Method according to Claim 1,
wherein the fixing of the carrier layer segment (TS) in the well (W) is performed on the base (WB) of the well (W) by means of an adhesive layer (K22, K22X) which is located between the well base (WB) and the carrier layer segment (TS).

9. Method according to Claim 1,
wherein the fixing of the carrier layer segment (TS) in the well (W2) is performed by means of jamming the carrier layer segment (TS) within the inner lateral wall (IS) of the well (WS).

10. Microtitration plate (MTX) having at least one well (W, W2) having capture molecular coatings,
wherein the capture molecules are antigens or antibodies,
having
- a carrier layer segment (TS) which is positioned and fixed above a well base in the well (W, W2);
- furthermore two or more carrier membrane segments (MS1, MS2) which are positioned and fixed on the carrier layer segment (TS) and which each comprise a plurality of blot line segments (LA11, ..., LA14, LA21, ..., LA23), wherein the respective blot line segments have respective capture molecule types.

11. Microtitration plate according to Claim 10,
wherein the blot line segments of a carrier membrane segment (MS1, MS2) are blot line segments which run so as to be mutually parallel.

12. Microtitration plate according to Claim 11, furthermore comprising, above the carrier layer segment (TS), at least one adhesive layer (K11, K22, K1X, K11X) on which the carrier membrane segments (MS1, MS2) are fixed.

13. Microtitration plate according to Claim 12,
wherein the adhesive layer is composed of a plurality of adhesive layer portions (K11, K21), and wherein the adhesive layer portions (K11, K21) are in each case portions of a double-sided adhesive film (K1).

14. Microtitration plate according to Claim 12,
wherein furthermore the carrier layer segment (TS) is fixed on the well base (WB) of the well (W) by means of a further adhesive layer (K22, K2X), wherein furthermore the further adhesive layer (K22) is preferably a double-sided adhesive film (K2).

15. Microtitration plate according to Claim 10,
wherein the carrier membrane segments (MS1, MS2) each comprise
- an elongate extent;
- furthermore a longitudinal axis (LAC1, LAC2) along the elongate extent; and
- furthermore a transverse axis (Q1, Q2) which is perpendicular to the longitudinal axis (LAC1, LAC2);
wherein furthermore the carrier layer segment (TS) has a circular base area (FL); and wherein furthermore the elongate carrier membrane segments (MS1, MS2) are positioned on the carrier layer segment (TS) in such a manner that
- the carrier membrane segments (MS1, MS2) by way of their respective longitudinal axis (LAC1, LAC2) are in each case positioned perpendicularly to a central axis (MA) of the circular base area (FL) of the carrier layer segment (TS);
- furthermore the first carrier membrane segment (MS1) by way of its transverse axis (Q1) is positioned so as to be offset from the central axis (MA) by a first distance (DI1); and
- furthermore the second carrier membrane segment (MS2) by way of its transverse axis (Q2) is positioned so as to be offset from the central axis (MA) by a second distance (DI2) which is different from the first distance (DI1).

16. Method comprising the following steps:
- providing a microtitration plate (MTX) according to one of Claims 10 to 15, comprising the carrier membrane segments (MS1, MS2);
- incubating the carrier membrane segments (MS1, MS2) with a patient sample, a conjugate and a substrate.

17. Method according to Claim 16,
furthermore comprising the following steps:
- detecting an image (B) which represents the carrier layer segment (TS) and the carrier membrane segments (MS1, MS2);
- identifying the first carrier membrane segment (MS1) and the second carrier membrane segment (MS2) in the image (B);
- determining respective measures of binding which indicate respective bindings of antibodies or antigens to respective blot line segments;
- issuing the respective measure of binding.

18. Method according to Claim 17,
wherein the microtitration plate is a microtitration plate according to Claim 15, furthermore comprising the following steps:
- providing a dataset (DA) which indicates respective membrane segment types for respective spatial orientations of respective transverse axes;
- identifying the first carrier membrane segment (MS1) and the second carrier membrane segment (MS2) in the image (B) while using the dataset;
- providing partial images (MSX11, MSX12) which represent the respective membrane segments (MS1, MS2).

## Revendications

1. Procédé de fabrication d'une plaque de microtitrage (MTX) avec des revêtements de molécules de capture,
dans lequel les molécules de capture sont des antigènes ou des anticorps,
comprenant
- la fourniture d'une première membrane de support (M1) ainsi que d'une deuxième membrane de support (M2),
- le revêtement de la première membrane de support (M1) avec des premières lignes de transfert respectives (BL1) présentant respectivement un type de molécule de capture respectif,
- le revêtement de la deuxième membrane de support (M2) avec des deuxièmes lignes de transfert respectives (BL2) présentant respectivement un type de molécule de capture respectif,
- la séparation d'un premier segment de membrane (MS1) de la première membrane de support (M1), qui présente des sections de ligne respectives (LA11, ..., LA14) des premières lignes de transfert respectives (BL1),
- la séparation d'un deuxième segment de membrane (MS2) de la deuxième membrane de support (M2), qui présente des sections de ligne respectives (LA21, ..., LA23) des deuxièmes lignes de transfert respectives (BL2),
- le positionnement et la fixation du premier segment de membrane (MS1) sur une couche de support (T),
- le positionnement et la fixation du deuxième segment de membrane (MS2) sur la couche de support (T),
- la séparation d'un segment de couche de support (TS) de la couche de support (T), qui présente le premier et le deuxième segment de membrane (MS1, MS2),
- le positionnement et la fixation du segment de couche de support (TS) dans un puits (W, W2) de la plaque de microtitrage (MTX).

2. Procédé selon la revendication 1,
dans lequel les premières lignes de transfert (BL1) sont des lignes de transfert s'étendant parallèlement entre elles, et dans lequel les deuxièmes lignes de transfert (BL2) sont des lignes de transfert s'étendant parallèlement entre elles.

3. Procédé selon la revendication 1,
comprenant en outre
- le blocage des premières lignes de transfert (BL1) sur la première membrane de support (M1) avec un premier tampon de blocage,
- ainsi que le blocage des deuxièmes lignes de transfert (BL2) sur la deuxième membrane de support (M2) avec un deuxième tampon de blocage.

4. Procédé selon la revendication 1,
dans lequel la séparation des segments de membrane (MS1, MS2) est effectuée par découpage, de préférence par poinçonnage.

5. Procédé selon la revendication 1,
dans lequel les segments de membrane (MS1, MS2) présentent respectivement
- une extension longitudinale,
- en outre, un axe longitudinal (LAC1, LAC2) le long de l'extension longitudinale,
- ainsi qu'en outre un axe transversal (Q1, Q2) perpendiculaire à l'axe longitudinal (LAC1, LAC2).

6. Procédé selon la revendication 5,
dans lequel le segment de couche de support (TS) présente une surface de base circulaire (FL),
dans lequel en outre le positionnement des segments de membrane (MS1, MS2) et la séparation du segment de couche de support (TS) sont effectués de telle sorte que les segments de membrane longitudinaux (MS1, MS2) sont positionnés sur le segment de couche de support (TS) de telle manière que
- les segments de membrane (MS1, MS2) sont positionnés avec leur axe longitudinal respectif (LAC1, LAC2) respectivement perpendiculairement à un axe central (MA) de la surface de base circulaire (FL) du segment de couche de support (TS),
- en outre, le premier segment de membrane (MS1) est positionné avec son axe transversal (Q1) décalé par rapport à l'axe central (MA) d'une première distance (DI1),
- et en outre, le deuxième segment de membrane (MS2) est positionné avec son axe transversal (Q2) décalé par rapport à l'axe central (MA) d'une deuxième distance (DI2) qui est différente de la première distance (DI1).

7. Procédé selon la revendication 1,
dans lequel la fixation des segments de membrane (MS1, MS2) sur la couche de support (T) est effectuée au moyen d'une couche adhésive (K11, K21, K1X, K11X) se trouvant entre les segments de membrane (MS1, MS2) et la couche de support (T).

8. Procédé selon la revendication 1,
dans lequel la fixation du segment de couche support (TS) dans le puits (W) sur le fond (WB) du puits (W) est effectuée au moyen d'une couche adhésive (K22, K22X) se trouvant entre le fond de puits (WB) et le segment de couche support (TS).

9. Procédé selon la revendication 1,
dans lequel la fixation du segment de couche de support (TS) dans le puits (W2) est effectuée par serrage du segment de couche de support (TS) à l'intérieur de la paroi latérale intérieure (IS) du puits (WS).

10. Plaque de microtitrage (MTX) avec au moins un puits (W, W2) avec des revêtements de molécules de capture,
dans laquelle les molécules de capture sont des antigènes ou des anticorps,
présentant
- un segment de couche de support (TS) positionné et fixé au-dessus d'un fond de puits dans le puits (W, W2),
- en outre deux ou plus de deux segments de membrane de support (MS1, MS2) positionnés et fixés sur le segment de couche de support (TS), présentant respectivement plusieurs segments de ligne de transfert (LA11, ..., LA14, LA21, ..., LA23), les segments de ligne de transfert respectifs présentant des types de molécules de capture respectifs.

11. Plaque de microtitrage selon la revendication 10,
dans laquelle les segments de ligne de transfert d'un segment de membrane de support (MS1, MS2) sont des segments de ligne de transfert s'étendant parallèlement entre eux.

12. Plaque de microtitrage selon la revendication 11,
comprenant en outre, au-dessus du segment de couche support (TS), au moins une couche adhésive (K11, K22, K1X, K11X) sur laquelle les segments de membrane de support (MS1, MS2) sont fixés.

13. Plaque de microtitrage selon la revendication 12,
dans laquelle la couche adhésive est constituée de plusieurs sections de couche adhésive (K11, K21) et dans laquelle les sections de couche adhésive (K11, K21) sont respectivement des sections d'un film adhésif double face (K1).

14. Plaque de microtitrage selon la revendication 12,
dans laquelle, en outre, le segment de couche de support (TS) est fixé sur le fond de puits (WB) du puits (W) au moyen d'une autre couche adhésive (K22, K2X),
dans laquelle, en outre, l'autre couche adhésive (K22) est de préférence un film adhésif double face (K2).

15. Plaque de microtitrage selon la revendication 10,
dans laquelle les segments de membrane de support (MS1, MS2) présentent respectivement
- une extension longitudinale,
- en outre, un axe longitudinal (LAC1, LAC2) le long de l'extension longitudinale,
- ainsi qu'en outre un axe transversal (Q1, Q2) perpendiculaire à l'axe longitudinal (LAC1, LAC2),
dans laquelle, en outre, le segment de couche de support (TS) présente une surface de base circulaire (FL),
et dans laquelle en outre les segments de membrane de support longitudinaux (MS1, MS2) sont positionnés sur le segment de couche de support (TS) de telle manière que
- les segments de membrane de support (MS1, MS2) sont positionnés avec leur axe longitudinal respectif (LAC1, LAC2) respectivement perpendiculairement à un axe central (MA) de la surface de base circulaire (FL) du segment de couche de support (TS),
- en outre, le premier segment de membrane de support (MS1) est positionné avec son axe transversal (Q1) décalé par rapport à l'axe central (MA) d'une première distance (DI1),
- et en outre, le deuxième segment de membrane de support (MS2) est positionné avec son axe transversal (Q2) décalé par rapport à l'axe central (MA) d'une deuxième distance (DI2) qui est différente de la première distance (DI1).

16. Procédé
comprenant
- la fourniture d'une plaque de microtitrage (MTX) selon l'une quelconque des revendications 10 à 15, présentant les segments de membrane de support (MS1, MS2),
- l'incubation des segments de membrane de support (MS1, MS2) avec un échantillon de patient, un conjugué et un substrat.

17. Procédé selon la revendication 16,
comprenant en outre
- l'acquisition d'une image (B) représentant le segment de couche de support (TS) et les segments de membrane de support (MS1, MS2),
- l'identification du premier segment de membrane de support (MS1) et du deuxième segment de membrane de support (MS2) dans l'image (B),
- la détermination de mesures de liaison respectives indiquant une liaison respective d'anticorps ou d'antigènes à des segments de ligne de transfert respectifs,
- la sortie des mesures de liaison respectives.

18. Procédé selon la revendication 17,
dans lequel la plaque de microtitrage est une plaque de microtitrage selon la revendication 15,
comprenant en outre
- la fourniture d'un ensemble de données (DA) indiquant des types de segments de membrane respectifs pour des positions spatiales respectives d'axes transversaux respectifs,
- l'identification du premier segment de membrane de support (MS1) et du deuxième segment de membrane de support (MS2) dans l'image (B) en utilisant l'ensemble de données (DA),
- la fourniture d'images partielles (MSX11, MSX12) représentant les segments de membrane respectifs (MS1, MS2).
